# EUROPÄISCHE PATENTSCHRIFT

(11) **EP 2 633 034 B1**
(45) Veröffentlichungstag und Bekanntmachung des Hinweises auf die Patenterteilung: **29.11.2017**
(21) Anmeldenummer: 11787599.7
(22) Anmeldetag: 26.10.2011
(51) Int. Cl.: C12N 5/0784, C12N 9/12, A61K 48/00, C12N 15/85, A61K 35/12, A61K 39/00

(54) **NFkB-SIGNALWEG-MANIPULIERTE DENDRITISCHE ZELLEN**
NFkB SIGNAL PATH-MANIPULATED DENDRITIC CELLS
CELLULES DENDRITIQUES À VOIE DE SIGNALISATION NFkB MANIPULÉE

(30) Priorität: 26.10.2010 EP 10188893
(43) Veröffentlichungstag der Anmeldung: 04.09.2013
(73) Patentinhaber: Friedrich-Alexander-Universität Erlangen-Nürnberg, 91054 Erlangen (DE)
(72) Erfinder: BIRKHOLZ, Katrin, 82069 Hohenschäftlarn (DE); DÖRRIE, Jan, 90411 Nürnberg (DE); SCHAFT, Niels, 91085 Weisendorf (DE); SCHULER, Gerold, 91080 Spardorf (DE); VOLL, Reinhard, 79114 Freiburg im Breisgau (DE); PFEIFFER, Isabell, 81369 München (DE)
(74) Vertreter: Vossius & Partner Patentanwälte Rechtsanwälte mbB
(86) Internationale Anmeldenummer: PCT/EP2011/005400
(87) Internationale Veröffentlichungsnummer: WO 2012/055551

(56) Entgegenhaltungen:
- EP-A1- 1 739 186
- WO-A2-2007/137300
- US-A1- 2009 202 492
- BOCZKOWSKI DAVID ET AL: "RNA as performance-enhancers for dendritic cells.", EXPERT OPINION ON BIOLOGICAL THERAPY APR 2010 LNKD- PUBMED:20128707, Bd. 10, Nr. 4, April 2010 (2010-04), Seiten 563-574, XP008134288, ISSN: 1744-7682
- KAISHO TSUNEYASU ET AL: "Turning NF-kappaB and IRFs on and off in DC.", TRENDS IN IMMUNOLOGY JUL 2008 LNKD- PUBMED:18534908, Bd. 29, Nr. 7, Juli 2008 (2008-07), Seiten 329-336, XP002627595, ISSN: 1471-4906
- SISTIANA AIELLO ET AL: "DnIKK2-Transfected Dendritic Cells Induce a Novel Population of Inducible Nitric Oxide Synthase Expressing CD4+CD25- Cells with Tolerogenic Properties", TRANSPLANTATION, Bd. 83, Nr. 4, 1. Februar 2007 (2007-02-01), Seiten 474-484, XP55025885, ISSN: 0041-1337, DOI: 10.1097/01.tp.0000251808.91901.c3
- SUSANNA TOMASONI ET AL: "Dendritic Cells Genetically Engineered with Adenoviral Vector Encoding dnIKK2 Induce the Formation of Potent CD4+ T-Regulatory Cells", TRANSPLANTATION, Bd. 79, Nr. 9, 1. Mai 2005 (2005-05-01), Seiten 1056-1061, XP55025883, ISSN: 0041-1337, DOI: 10.1097/01.TP.0000161252.17163.31
- DELHASE M ET AL: "Positive and negative regulation of IkB kinase activity through IKKB subunit phosphorylation", SCIENCE, AMERICAN ASSOCIATION FOR THE ADVANCEMENT OF SCIENCE, US, vol. 284, 9 April 1999 (1999-04-09), pages 309-313, XP002189522, ISSN: 0036-8075, DOI: 10.1126/SCIENCE.284.5412.309

### SEQUENCE LISTING

<110> Friedrich-Alexander-universität Erlangen-Nürnberg
<120> NFkappaB-Signalweg-manipulierte dendritische Zellen
<130> T3091 PCT
<150> EP 10 18 8893.1
   <151> 2010-10-26
<160> 10
<170> PatentIn version 3.3
<210> 1
   <211> 745
   <212> PRT
   <213> Homo sapiens
<400> 1
   Met Glu Arg Pro Pro Gly Leu Arg Pro Gly Ala Gly Gly Pro Trp Glu
   1               5                   10                  15
   Met Arg Glu Arg Leu Gly Thr Gly Gly Phe Gly Asn Val Cys Leu Tyr
               20                  25                  30
   Gln His Arg Glu Leu Asp Leu Lys Ile Ala Ile Lys Ser Cys Arg Leu
           35                  40                  45
   Glu Leu Ser Thr Lys Asn Arg Glu Arg Trp Cys His Glu Ile Gln Ile
       50                  55                  60
   Met Lys Lys Leu Asn His Ala Asn Val Val Lys Ala Cys Asp Val Pro
   65                  70                  75                  80
   Glu Glu Leu Asn Ile Leu Ile His Asp Val Pro Leu Leu Ala Met Glu
                   85                  90                  95
   Tyr Cys Ser Gly Gly Asp Leu Arg Lys Leu Leu Asn Lys Pro Glu Asn
               100                 105                 110
   Cys Cys Gly Leu Lys Glu Ser Gln Ile Leu Ser Leu Leu Ser Asp Ile
           115                 120                 125
   Gly Ser Gly Ile Arg Tyr Leu His Glu Asn Lys Ile Ile His Arg Asp
       130                 135                 140
   Leu Lys Pro Glu Asn Ile Val Leu Gln Asp Val Gly Gly Lys Ile Ile
   145                 150                 155                 160
   His Lys Ile Ile Asp Leu Gly Tyr Ala Lys Asp Val Asp Gln Gly Ser
                   165                 170                 175
   Leu Cys Thr Ser Phe Val Gly Thr Leu Gln Tyr Leu Ala Pro Glu Leu
               180                 185                 190
   Phe Glu Asn Lys Pro Tyr Thr Ala Thr Val Asp Tyr Trp Ser Phe Gly
           195                 200                 205
   Thr Met Val Phe Glu Cys Ile Ala Gly Tyr Arg Pro Phe Leu His His
       210                 215                 220
   Leu Gln Pro Phe Thr Trp His Glu Lys Ile Lys Lys Lys Asp Pro Lys
   225                 230                 235                 240
   Cys Ile Phe Ala Cys Glu Glu Met Ser Gly Glu Val Arg Phe Ser Ser
                   245                 250                 255
   His Leu Pro Gln Pro Asn Ser Leu Cys Ser Leu Ile Val Glu Pro Met
               260                 265                 270
   Glu Asn Trp Leu Gln Leu Met Leu Asn Trp Asp Pro Gln Gln Arg Gly
           275                 280                 285
   Gly Pro Val Asp Leu Thr Leu Lys Gln Pro Arg Cys Phe Val Leu Met
       290                 295                 300
   Asp His Ile Leu Asn Leu Lys Ile Val His Ile Leu Asn Met Thr Ser
   305                 310                 315                 320
   Ala Lys Ile Ile Ser Phe Leu Leu Pro Pro Asp Glu Ser Leu His Ser
                   325                 330                 335
   Leu Gln Ser Arg Ile Glu Arg Glu Thr Gly Ile Asn Thr Gly Ser Gln
               340                 345                 350
   Glu Leu Leu Ser Glu Thr Gly Ile Ser Leu Asp Pro Arg Lys Pro Ala
           355                 360                 365
   Ser Gln Cys Val Leu Asp Gly Val Arg Gly Cys Asp Ser Tyr Met Val
       370                 375                 380
   Tyr Leu Phe Asp Lys Ser Lys Thr Val Tyr Glu Gly Pro Phe Ala Ser
   385                 390                 395                 400
   Arg Ser Leu Ser Asp Cys Val Asn Tyr Ile Val Gln Asp Ser Lys Ile
                   405                 410                 415
   Gln Leu Pro Ile Ile Gln Leu Arg Lys Val Trp Ala Glu Ala Val His
               420                 425                 430
   Tyr Val Ser Gly Leu Lys Glu Asp Tyr Ser Arg Leu Phe Gln Gly Gln
           435                 440                 445
   Arg Ala Ala Met Leu Ser Leu Leu Arg Tyr Asn Ala Asn Leu Thr Lys
       450                 455                 460
   Met Lys Asn Thr Leu Ile Ser Ala Ser Gln Gln Leu Lys Ala Lys Leu
   465 470 475 480
   Glu Phe Phe His Lys Ser Ile Gln Leu Asp Leu Glu Arg Tyr Ser Glu
   485 490 495
   Gln Met Thr Tyr Gly Ile Ser Ser Glu Lys Met Leu Lys Ala Trp Lys
   500 505 510
   Glu Met Glu Glu Lys Ala Ile His Tyr Ala Glu Val Gly Val Ile Gly
   515 520 525
   Tyr Leu Glu Asp Gln Ile Met Ser Leu His Ala Glu Ile Met Glu Leu
   530 535 540
   Gln Lys Ser Pro Tyr Gly Arg Arg Gln Gly Asp Leu Met Glu Ser Leu
   545 550 555 560
   Glu Gln Arg Ala Ile Asp Leu Tyr Lys Gln Leu Lys His Arg Pro Ser
   565 570 575
   Asp His Ser Tyr Ser Asp Ser Thr Glu Met Val Lys Ile Ile Val His
   580 585 590
   Thr Val Gln Ser Gln Asp Arg Val Leu Lys Glu Leu Phe Gly His Leu
   595 600 605
   Ser Lys Leu Leu Gly Cys Lys Gln Lys Ile Ile Asp Leu Leu Pro Lys
   610 615 620
   Val Glu Val Ala Leu Ser Asn Ile Lys Glu Ala Asp Asn Thr Val Met
   625 630 635 640
   Phe Met Gln Gly Lys Arg Gln Lys Glu Ile Trp His Leu Leu Lys Ile
   645 650 655
   Ala Cys Thr Gln Ser Ser Ala Arg Ser Leu Val Gly Ser Ser Leu Glu
   660 665 670
   Gly Ala Val Thr Pro Gln Thr Ser Ala Trp Leu Pro Pro Thr Ser Ala
   675 680 685
   Glu His Asp His Ser Leu Ser Cys Val Val Thr Pro Gln Asp Gly Glu
   690 695 700
   Thr Ser Ala Gln Met Ile Glu Glu Asn Leu Asn Cys Leu Gly His Leu
   705 710 715 720
   Ser Thr Ile Ile His Glu Ala Asn Glu Glu Gln Gly Asn Ser Met Met
   725 730 735
   Asn Leu Asp Trp Ser Trp Leu Thr Glu
   740 745
<210> 2
   <211> 769
   <212> PRT
   <213> Artificial
<220>
   <223> IKKalpha konstitutiv aktive Mutante
<400> 2
   His His His His His Gly Asp Tyr Lys Asp Asp Asp Asp Lys Gly Asp
   1 5 10 15
   Ile Glu Gly Arg Gly His Met Thr Met Glu Arg Pro Pro Gly Leu Arg
   20 25 30
   Pro Gly Ala Gly Gly Pro Trp Glu Met Arg Glu Arg Leu Gly Thr Gly
   35 40 45
   Gly Phe Gly Asn Val Cys Leu Tyr Gln His Arg Glu Leu Asp Leu Lys
   50 55 60
   Ile Ala Ile Lys Ser Cys Arg Leu Glu Leu Ser Thr Lys Asn Arg Glu
   65 70 75 80
   Arg Trp Cys His Glu Ile Gln Ile Met Lys Lys Leu Asn His Ala Asn
   85 90 95
   Val Val Lys Ala Cys Asp Val Pro Glu Glu Leu Asn Ile Leu Ile His
   100 105 110
   Asp Val Pro Leu Leu Ala Met Glu Tyr Cys Ser Gly Gly Asp Leu Arg
   115 120 125
   Lys Leu Leu Asn Lys Pro Glu Asn Cys Cys Gly Leu Lys Glu Ser Gln
   130 135 140
   Ile Leu Ser Leu Leu Ser Asp Ile Gly Ser Gly Ile Arg Tyr Leu His
   145 150 155 160
   Glu Asn Lys Ile Ile His Arg Asp Leu Lys Pro Glu Asn Ile Val Leu
   165 170 175
   Gln Asp Val Gly Gly Lys Ile Ile His Lys Ile Ile Asp Leu Gly Tyr
   180 185 190
   Ala Lys Asp Val Asp Gln Gly Glu Leu Cys Thr Glu Phe Val Gly Thr
   195 200 205
   Leu Gln Tyr Leu Ala Pro Glu Leu Phe Glu Asn Lys Pro Tyr Thr Ala
   210 215 220
   Thr Val Asp Tyr Trp Ser Phe Gly Thr Met Val Phe Glu Cys Ile Ala
   225 230 235 240
   Gly Tyr Arg Pro Phe Leu His His Leu Gln Pro Phe Thr Trp His Glu
   245 250 255
   Lys Ile Lys Lys Lys Asp Pro Lys Cys Ile Phe Ala Cys Glu Glu Met
   260 265 270
   Ser Gly Glu Val Arg Phe Ser Ser His Leu Pro Gln Pro Asn Ser Leu
   275 280 285
   Cys Ser Leu Ile Val Glu Pro Met Glu Asn Trp Leu Gln Leu Met Leu
   290 295 300
   Asn Trp Asp Pro Gln Gln Arg Gly Gly Pro Val Asp Leu Thr Leu Lys
   305 310 315 320
   Gln Pro Arg Cys Phe Val Leu Met Asp His Ile Leu Asn Leu Lys Ile
   325 330 335
   Val His Ile Leu Asn Met Thr Ser Ala Lys Ile Ile Ser Phe Leu Leu
   340 345 350
   Pro Pro Asp Glu Ser Leu His Ser Leu Gln Ser Arg Ile Glu Arg Glu
   355 360 365
   Thr Gly Ile Asn Thr Gly Ser Gln Glu Leu Leu Ser Glu Thr Gly Ile
   370 375 380
   Ser Leu Asp Pro Arg Lys Pro Ala Ser Gln Cys Val Leu Asp Gly Val
   385 390 395 400
   Arg Gly Cys Asp Ser Tyr Met Val Tyr Leu Phe Asp Lys Ser Lys Thr
   405 410 415
   Val Tyr Glu Gly Pro Phe Ala Ser Arg Ser Leu Ser Asp Cys Val Asn
   420 425 430
   Tyr Ile Val Gln Asp Ser Lys Ile Gln Leu Pro Ile Ile Gln Leu Arg
   435 440 445
   Lys Ala Trp Ala Glu Ala Val His Tyr Val Ser Gly Leu Lys Glu Asp
   450 455 460
   Tyr Ser Arg Leu Phe Gln Gly Gln Arg Ala Ala Met Leu Ser Leu Leu
   465 470 475 480
   Arg Tyr Asn Ala Asn Leu Thr Lys Met Lys Asn Thr Leu Ile Ser Ala
   485 490 495
   Ser Gln Gln Leu Lys Ala Lys Leu Glu Phe Phe His Lys Ser Ile Gln
   500 505 510
   Leu Asp Leu Glu Arg Tyr Ser Glu Gln Met Thr Tyr Gly Ile Ser Ser
   515 520 525
   Glu Lys Met Leu Lys Ala Trp Lys Glu Met Glu Glu Lys Ala Ile His
   530 535 540
   Tyr Ala Glu Val Gly Val Ile Gly Tyr Leu Glu Asp Gln Ile Met Ser
   545 550 555 560
   Leu His Ala Glu Ile Met Glu Leu Gln Lys Ser Pro Tyr Gly Arg Arg
   565 570 575
   Gln Gly Asp Leu Met Glu Ser Leu Glu Gln Arg Ala Ile Asp Leu Tyr
   580 585 590
   Lys Gln Leu Lys His Arg Pro Ser Asp His Ser Tyr Ser Asp Ser Thr
   595 600 605
   Glu Met Val Lys Ile Ile Val His Thr Val Gln Ser Gln Asp Arg Val
   610 615 620
   Leu Lys Glu Leu Phe Gly His Leu Ser Lys Leu Leu Gly Cys Lys Gln
   625 630 635 640
   Lys Ile Ile Asp Leu Leu Pro Lys Val Glu Val Ala Leu Ser Asn Ile
   645 650 655
   Lys Glu Ala Asp Asn Thr Val Met Phe Met Gln Gly Lys Arg Gln Lys
   660 665 670
   Glu Ile Trp His Leu Leu Lys Ile Ala Cys Thr Gln Ala Ala Ala Arg
   675 680 685
   Ala Leu Val Gly Ala Ala Leu Glu Gly Ala Val Ala Pro Gln Ala Ala
   690 695 700
   Ala Trp Leu Pro Pro Ala Ala Ala Glu His Asp His Ala Leu Ala Cys
   705 710 715 720
   Val Val Ala Pro Gln Asp Gly Glu Ala Ala Ala Gln Met Ile Glu Glu
   725 730 735
   Asn Leu Asn Cys Leu Gly His Leu Ala Ala Ile Ile His Glu Ala Asn
   740 745 750
   Glu Glu Gln Gly Asn Ser Met Met Asn Leu Asp Trp Ser Trp Leu Thr
   755 760 765
   Glu
<210> 3
   <211> 2238
   <212> DNA
   <213> Artificial
<220>
   <223> IKKalpha konstitutiv aktive Mutante
<400> 3
   atggagcggc ccccggggct gcggccgggc gcgggcgggc cctgggagat gcgggagcgg 60
   ctgggcaccg gcggcttcgg gaacgtctgt ctgtaccagc atcgggaact tgatctcaaa 120
   atagcaatta agtcttgtcg cctagagcta agtaccaaaa acagagaacg atggtgccat 180
   gaaatccaga ttatgaagaa gttgaaccat gccaatgttg taaaggcctg tgatgttcct 240
   gaagaattga atattttgat tcatgatgtg cctcttctag caatggaata ctgttctgga 300
   ggagatctcc gaaagctgct caacaaacca gaaaattgtt gtggacttaa agaaagccag 360
   atactttctt tactaagtga tatagggtct gggattcgat atttgcatga aaacaaaatt 420
   atacatcgag atctaaaacc tgaaaacata gttcttcagg atgttggtgg aaagataata 480
   cataaaataa ttgatctggg atatgccaaa gatgttgatc aaggagagct gtgtacagag 540
   tttgtgggaa cactgcagta tctggcccca gagctctttg agaataagcc ttacacagcc 600
   actgttgatt attggagctt tgggaccatg gtatttgaat gtattgctgg atataggcct 660
   tttttgcatc atctgcagcc atttacctgg catgagaaga ttaagaagaa ggatccaaag 720
   tgtatatttg catgtgaaga gatgtcagga gaagttcggt ttagtagcca tttacctcaa 780
   ccaaatagcc tttgtagttt aatagtagaa cccatggaaa actggctaca gttgatgttg 840
   aattgggacc ctcagcagag aggaggacct gttgacctta ctttgaagca gccaagatgt 900
   tttgtattaa tggatcacat tttgaatttg aagatagtac acatcctaaa tatgacttct 960
   gcaaagataa tttcttttct gttaccacct gatgaaagtc ttcattcatt acagtctcgt 1020
   attgagcgtg aaactggaat aaatactggt tctcaagaac ttctttcaga gacaggaatt 1080
   tctctggatc ctcggaaacc agcctctcaa tgtgttctag atggagttag aggctgtgat 1140
   agctatatgg tttatttgtt tgataaaagt aaaactgtat atgaagggcc atttgcttcc 1200
   agaagtttat ctgattgtgt aaattatatt gtacaggaca gcaaaataca gcttccaatt 1260
   atacagctgc gtaaagcgtg ggctgaagca gtgcactatg tgtctggact aaaagaagac 1320
   tatagcaggc tctttcaggg acaaagggca gcaatgttaa gtcttcttag atataatgct 1380
   aacttaacaa aaatgaagaa cactttgatc tcagcatcac aacaactgaa agctaaattg 1440
   gagttttttc acaaaagcat tcagcttgac ttggagagat acagcgagca gatgacgtat 1500
   gggatatctt cagaaaaaat gctaaaagca tggaaagaaa tggaagaaaa ggccatccac 1560
   tatgctgagg ttggtgtcat tggatacctg gaggatcaga ttatgtcttt gcatgctgaa 1620
   atcatggagc tacagaagag cccctatgga agacgtcagg gagacttgat ggaatctctg 1680
   gaacagcgtg ccattgatct atataagcag ttaaaacaca gaccttcaga tcactcctac 1740
   agtgacagca cagagatggt gaaaatcatt gtgcacactg tgcagagtca ggaccgtgtg 1800
   ctcaaggagc tgtttggtca tttgagcaag ttgttgggct gtaagcagaa gattattgat 1860
   ctactcccta aggtggaagt ggccctcagt aatatcaaag aagctgacaa tactgtcatg 1920
   ttcatgcagg gaaaaaggca gaaagaaatc tggcatctcc ttaaaattgc ctgtacacag 1980
   gccgctgccc gcgcccttgt gggagccgct ctggaaggtg cagtggcccc acaggccgcc 2040
   gcatggctgc cccctgctgc cgcagaacac gatcacgctc tggcctgtgt ggtggctcct 2100
   caagatgggg aggctgccgc acaaatgatc gaagaaaatt tgaactgcct tggccacttg 2160
   gccgctatta ttcacgaggc aaatgaggaa cagggcaata gtatgatgaa tcttgattgg 2220
   agttggttga cagaatga 2238
<210> 4
   <211> 756
   <212> PRT
   <213> Homo sapiens
<400> 4
<210> 5
   <211> 773
   <212> PRT
   <213> Artificial
<220>
   <223> IKKbeta konstitutiv aktive Mutante
<400> 5
   Met Asp Tyr Lys Asp Asp Asp Asp Lys Gly Asp Ile Glu Gly Arg Gly
   1               5                   10                  15
   His Met Ser Trp Ser Pro Ser Leu Thr Thr Gln Thr Cys Gly Ala Trp
               20                  25                  30
   Glu Met Lys Glu Arg Leu Gly Thr Gly Gly Phe Gly Asn Val Ile Arg
           35                  40                  45
   Trp His Asn Gln Glu Thr Gly Glu Gln Ile Ala Ile Lys Gln Cys Arg
       50                  55                  60
   Gln Glu Leu Ser Pro Arg Asn Arg Glu Arg Trp Cys Leu Glu Ile Gln
   65                  70                  75                  80
   Ile Met Arg Arg Leu Thr His Pro Asn Val Val Ala Ala Arg Asp Val
                   85                  90                  95
   Pro Glu Gly Met Gln Asn Leu Ala Pro Asn Asp Leu Pro Leu Leu Ala
               100                 105                 110
   Met Glu Tyr Cys Gln Gly Gly Asp Leu Arg Lys Tyr Leu Asn Gln Phe
           115                 120                 125
   Glu Asn Cys Cys Gly Leu Arg Glu Gly Ala Ile Leu Thr Leu Leu Ser
       130                 135                 140
   Asp Ile Ala Ser Ala Leu Arg Tyr Leu His Glu Asn Arg Ile Ile His
   145 150 155 160
   Arg Asp Leu Lys Pro Glu Asn Ile Val Leu Gln Gln Gly Glu Gln Arg
   165 170 175
   Leu Ile His Lys Ile Ile Asp Leu Gly Tyr Ala Lys Glu Leu Asp Gln
   180 185 190
   Gly Glu Leu Cys Thr Glu Phe Val Gly Thr Leu Gln Tyr Leu Ala Pro
   195 200 205
   Glu Leu Leu Glu Gln Gln Lys Tyr Thr Val Thr Val Asp Tyr Trp Ser
   210 215 220
   Phe Gly Thr Leu Ala Phe Glu Cys Ile Thr Gly Phe Arg Pro Phe Leu
   225 230 235 240
   Pro Asn Trp Gln Pro Val Gln Trp His Ser Lys Val Arg Gln Lys Ser
   245 250 255
   Glu Val Asp Ile Val Val Ser Glu Asp Leu Asn Gly Thr Val Lys Phe
   260 265 270
   Ser Ser Ser Leu Pro Tyr Pro Asn Asn Leu Asn Ser Val Leu Ala Glu
   275 280 285
   Arg Leu Glu Lys Trp Leu Gln Leu Met Leu Met Trp His Pro Arg Gln
   290 295 300
   Arg Gly Thr Asp Pro Thr Tyr Gly Pro Asn Gly Cys Phe Lys Ala Leu
   305 310 315 320
   Asp Asp Ile Leu Asn Leu Lys Leu Val His Ile Leu Asn Met Val Thr
   325 330 335
   Gly Thr Ile His Thr Tyr Pro Val Thr Glu Asp Glu Ser Leu Gln Ser
   340 345 350
   Leu Lys Ala Arg Ile Gln Gln Asp Thr Gly Ile Pro Glu Glu Asp Gln
   355 360 365
   Glu Leu Leu Gln Glu Ala Gly Leu Ala Leu Ile Pro Asp Lys Pro Ala
   370 375 380
   Thr Gln Cys Ile Ser Asp Gly Lys Leu Asn Glu Gly His Thr Leu Asp
   385 390 395 400
   Met Asp Leu Val Phe Leu Phe Asp Asn Ser Lys Ile Thr Tyr Glu Thr
   405 410 415
   Gln Ile Ser Pro Arg Pro Gln Pro Glu Ser Val Ser Cys Ile Leu Gln
   420 425 430
   Glu Pro Lys Arg Asn Leu Ala Phe Phe Gln Leu Arg Lys Val Trp Gly
   435 440 445
   Gln Val Trp His Ser Ile Gln Thr Leu Lys Glu Asp Cys Asn Arg Leu
   450 455 460
   Gln Gln Gly Gln Arg Ala Ala Met Met Asn Leu Leu Arg Asn Asn Ser
   465 470 475 480
   Cys Leu Ser Lys Met Lys Asn Ser Met Ala Ser Met Ser Gln Gln Leu
   485 490 495
   Lys Ala Lys Leu Asp Phe Phe Lys Thr Ser Ile Gln Ile Asp Leu Glu
   500 505 510
   Lys Tyr Ser Glu Gln Thr Glu Phe Gly Ile Thr Ser Asp Lys Leu Leu
   515 520 525
   Leu Ala Trp Arg Glu Met Glu Gln Ala Val Glu Leu Cys Gly Arg Glu
   530 535 540
   Asn Glu Val Lys Leu Leu Val Glu Arg Met Met Ala Leu Gln Thr Asp
   545 550 555 560
   Ile Val Asp Leu Gln Arg Ser Pro Met Gly Arg Lys Gln Gly Gly Thr
   565 570 575
   Leu Asp Asp Leu Glu Glu Gln Ala Arg Glu Leu Tyr Arg Arg Leu Arg
   580 585 590
   Glu Lys Pro Arg Asp Gln Arg Thr Glu Gly Asp Ser Gln Glu Met Val
   595 600 605
   Arg Leu Leu Leu Gln Ala Ile Gln Ser Phe Glu Lys Lys Val Arg Val
   610 615 620
   Ile Tyr Thr Gln Leu Ser Lys Thr Val Val Cys Lys Gln Lys Ala Leu
   625 630 635 640
   Glu Leu Leu Pro Lys Val Glu Glu Val Val Ser Leu Met Asn Glu Asp
   645 650 655
   Glu Lys Thr Val Val Arg Leu Gln Glu Lys Arg Gln Lys Glu Leu Trp
   660 665 670
   Asn Leu Leu Lys Ile Ala Cys Ser Lys Val Arg Gly Pro Val Ala Gly
   675 680 685
   Ala Pro Asp Ala Met Asn Ala Ala Arg Leu Ala Gln Pro Gly Gln Leu
   690 695 700
   Met Ala Gln Pro Ala Thr Ala Ala Asn Ala Leu Pro Glu Pro Ala Lys
   705 710 715 720
   Lys Ala Glu Glu Leu Val Ala Glu Ala His Asn Leu Cys Thr Leu Leu
   725 730 735
   Glu Asn Ala Ile Gln Asp Thr Val Arg Glu Gln Asp Gln Ser Phe Thr
   740 745 750
   Ala Leu Asp Trp Ser Trp Leu Gln Thr Glu Glu Glu Glu His Ser Cys
   755 760 765
   Leu Glu Gln Ala Ser
   770
<210> 6
   <211> 2319
   <212> DNA
   <213> Artificial
<220>
   <223> IKKbeta konstitutiv aktive Mutante
<400> 6
   atggactaca aggacgacga tgacaaaggt gacatcgaag gtagaggtca tatgagctgg 60
   tcaccttccc tgacaacgca gacatgcggg gcctgggaaa tgaaagagcg ccttgggaca 120
   gggggatttg gaaatgtcat ccgatggcac aatcaggaaa caggtgagca gattgccatc 180
   aagcagtgcc ggcaggagct cagcccccgg aaccgagagc ggtggtgcct ggagatccag 240
   atcatgagaa ggctgaccca ccccaatgtg gtggctgccc gagatgtccc tgaggggatg 300
   cagaacttgg cgcccaatga cctgcccctg ctggccatgg agtactgcca aggaggagat 360
   ctccggaagt acctgaacca gtttgagaac tgctgtggtc tgcgggaagg tgccatcctc 420
   accttgctga gtgacattgc ctctgcgctt agataccttc atgaaaacag aatcatccat 480
   cgggatctaa agccagaaaa catcgtcctg cagcaaggag aacagaggtt aatacacaaa 540
   attattgacc taggatatgc caaggagctg gatcagggcg agctttgcac agagttcgtg 600
   gggaccctgc agtacctggc cccagagcta ctggagcagc agaagtacac agtgaccgtc 660
   gactactgga gcttcggcac cctggccttt gagtgcatca cgggcttccg gcccttcctc 720
   cccaactggc agcccgtgca gtggcattca aaagtgcggc agaagagtga ggtggacatt 780
   gttgttagcg aagacttgaa tggaacggtg aagttttcaa gctctttacc ctaccccaat 840
   aatcttaaca gtgtcctggc tgagcgactg gagaagtggc tgcaactgat gctgatgtgg 900
   cacccccgac agaggggcac ggatcccacg tatgggccca atggctgctt caaggccctg 960
   gatgacatct taaacttaaa gctggttcat atcttgaaca tggtcacggg caccatccac 1020
   acctaccctg tgacagagga tgagagtctg cagagcttga aggccagaat ccaacaggac 1080
   acgggcatcc cagaggagga ccaggagctg ctgcaggaag cgggcctggc gttgatcccc   1140
   gataagcctg ccactcagtg tatttcagac ggcaagttaa atgagggcca cacattggac   1200
   atggatcttg tttttctctt tgacaacagt aaaatcacct atgagactca gatctcccca   1260
   cggccccaac ctgaaagtgt cagctgtatc cttcaagagc ccaagaggaa tctcgccttc   1320
   ttccagctga ggaaggtgtg gggccaggtc tggcacagca tccagaccct gaaggaagat   1380
   tgcaaccggc tgcagcaggg acagcgagcc gccatgatga atctcctccg aaacaacagc   1440
   tgcctctcca aaatgaagaa ttccatggct tccatgtctc agcagctcaa ggccaagttg   1500
   gatttcttca aaaccagcat ccagattgac ctggagaagt acagcgagca aaccgagttt   1560
   gggatcacat cagataaact gctgctggcc tggagggaaa tggagcaggc tgtggagctc   1620
   tgtgggcggg agaacgaagt gaaactcctg gtagaacgga tgatggctct gcagaccgac   1680
   attgtggact tacagaggag ccccatgggc cggaagcagg ggggaacgct ggacgaccta   1740
   gaggagcaag caagggagct gtacaggaga ctaagggaaa aacctcgaga ccagcgaact   1800
   gagggtgaca gtcaggaaat ggtacggctg ctgcttcagg caattcagag cttcgagaag   1860
   aaagtgcgag tgatctatac gcagctcagt aaaactgtgg tttgcaagca gaaggcgctg   1920
   gaactgttgc ccaaggtgga agaggtggtg agcttaatga atgaggatga gaagactgtt   1980
   gtccggctgc aggagaagcg gcagaaggag ctctggaatc tcctgaagat tgcttgtagc   2040
   aaggtccgtg gtcctgtcgc tggagccccg gatgccatga atgccgctcg acttgcccag   2100
   cctgggcagc tgatggctca gcccgccacg gccgccaacg ccttacctga gccagccaag   2160
   aaggctgaag aactggtggc tgaagcacat aacctctgca ccctgctaga aaatgccata   2220
   caggacactg tgagggaaca agaccagagt ttcacggccc tagactggag ctggttacag   2280
   acggaagaag aagagcacag ctgcctggag caggcctca                          2319
<210> 7
   <211> 768
   <212> PRT
   <213> Artificial
<220>
   <223> IKKalpha hemmende Mutante
<400> 7
   His His His His Gly Asp Tyr Lys Asp Asp Asp Asp Lys Gly Asp Ile
   1               5                   10                  15
   Glu Gly Arg Gly His Met Thr Met Glu Arg Pro Pro Gly Leu Arg Pro
               20                  25                  30
   Gly Ala Gly Gly Pro Trp Glu Met Arg Glu Arg Leu Gly Thr Gly Gly
           35                  40                  45
   Phe Gly Asn Val Cys Leu Tyr Gln His Arg Glu Leu Asp Leu Lys Ile
       50                  55                  60
   Ala Ile Met Ser Cys Arg Leu Glu Leu Ser Thr Lys Asn Arg Glu Arg
   65 70 75 80
   Trp Cys His Glu Ile Gln Ile Met Lys Lys Leu Asn His Ala Asn Val
   85 90 95
   Val Lys Ala Cys Asp Val Pro Glu Glu Leu Asn Ile Leu Ile His Asp
   100 105 110
   Val Pro Leu Leu Ala Met Glu Tyr Cys Ser Gly Gly Asp Leu Arg Lys
   115 120 125
   Leu Leu Asn Lys Pro Glu Asn Cys Cys Gly Leu Lys Glu Ser Gln Ile
   130 135 140
   Leu Ser Leu Leu Ser Asp Ile Gly Ser Gly Ile Arg Tyr Leu His Glu
   145 150 155 160
   Asn Lys Ile Ile His Arg Asp Leu Lys Pro Glu Asn Ile Val Leu Gln
   165 170 175
   Asp Val Gly Gly Lys Ile Ile His Lys Ile Ile Asp Leu Gly Tyr Ala
   180 185 190
   Lys Asp Val Asp Gln Gly Ser Leu Cys Thr Ser Phe Val Gly Thr Leu
   195 200 205
   Gln Tyr Leu Ala Pro Glu Leu Phe Glu Asn Lys Pro Tyr Thr Ala Thr
   210 215 220
   Val Asp Tyr Trp Ser Phe Gly Thr Met Val Phe Glu Cys Ile Ala Gly
   225 230 235 240
   Tyr Arg Pro Phe Leu His His Leu Gln Pro Phe Thr Trp His Glu Lys
   245 250 255
   Ile Lys Lys Lys Asp Pro Lys Cys Ile Phe Ala Cys Glu Glu Met Ser
   260 265 270
   Gly Glu Val Arg Phe Ser Ser His Leu Pro Gln Pro Asn Ser Leu Cys
   275 280 285
   Ser Leu Ile Val Glu Pro Met Glu Asn Trp Leu Gln Leu Met Leu Asn
   290 295 300
   Trp Asp Pro Gln Gln Arg Gly Gly Pro Val Asp Leu Thr Leu Lys Gln
   305 310 315 320
   Pro Arg Cys Phe Val Leu Met Asp His Ile Leu Asn Leu Lys Ile Val
   325 330 335
   His Ile Leu Asn Met Thr Ser Ala Lys Ile Ile Ser Phe Leu Leu Pro
   340 345 350
   Pro Asp Glu Ser Leu His Ser Leu Gln Ser Arg Ile Glu Arg Glu Thr
   355 360 365
   Gly Ile Asn Thr Gly Ser Gln Glu Leu Leu Ser Glu Thr Gly Ile Ser
   370 375 380
   Leu Asp Pro Arg Lys Pro Ala Ser Gln Cys Val Leu Asp Gly Val Arg
   385 390 395 400
   Gly Cys Asp Ser Tyr Met Val Tyr Leu Phe Asp Lys Ser Lys Thr Val
   405 410 415
   Tyr Glu Gly Pro Phe Ala Ser Arg Ser Leu Ser Asp Cys Val Asn Tyr
   420 425 430
   Ile Val Gln Asp Ser Lys Ile Gln Leu Pro Ile Ile Gln Leu Arg Lys
   435 440 445
   Ala Trp Ala Glu Ala Val His Tyr Val Ser Gly Leu Lys Glu Asp Tyr
   450 455 460
   Ser Arg Leu Phe Gln Gly Gln Arg Ala Ala Met Leu Ser Leu Leu Arg
   465 470 475 480
   Tyr Asn Ala Asn Leu Thr Lys Met Lys Asn Thr Leu Ile Ser Ala Ser
   485 490 495
   Gln Gln Leu Lys Ala Lys Leu Glu Phe Phe His Lys Ser Ile Gln Leu
   500 505 510
   Asp Leu Glu Arg Tyr Ser Glu Gln Met Thr Tyr Gly Ile Ser Ser Glu
   515 520 525
   Lys Met Leu Lys Ala Trp Lys Glu Met Glu Glu Lys Ala Ile His Tyr
   530 535 540
   Ala Glu Val Gly Val Ile Gly Tyr Leu Glu Asp Gln Ile Met Ser Leu
   545 550 555 560
   His Ala Glu Ile Met Glu Leu Gln Lys Ser Pro Tyr Gly Arg Arg Gln
   565 570 575
   Gly Asp Leu Met Glu Ser Leu Glu Gln Arg Ala Ile Asp Leu Tyr Lys
   580 585 590
   Gln Leu Lys His Arg Pro Ser Asp His Ser Tyr Ser Asp Ser Thr Glu
   595 600 605
   Met Val Lys Ile Ile Val His Thr Val Gln Ser Gln Asp Arg Val Leu
       610                 615                 620
   Lys Glu Leu Phe Gly His Leu Ser Lys Leu Leu Gly Cys Lys Gln Lys
   625                 630                 635                 640
   Ile Ile Asp Leu Leu Pro Lys Val Glu Val Ala Leu Ser Asn Ile Lys
                   645                 650                 655
   Glu Ala Asp Asn Thr Val Met Phe Met Gln Gly Lys Arg Gln Lys Glu
               660                 665                 670
   Ile Trp His Leu Leu Lys Ile Ala Cys Thr Gln Ala Ala Ala Arg Ala
           675                 680                 685
   Leu Val Gly Ala Ala Leu Glu Gly Ala Val Ala Pro Gln Ala Ala Ala
       690                 695                 700
   Trp Leu Pro Pro Ala Ala Ala Glu His Asp His Ala Leu Ala Cys Val
   705                 710                 715                 720
   Val Ala Pro Gln Asp Gly Glu Ala Ala Ala Gln Met Ile Glu Glu Asn
                   725                 730                 735
   Leu Asn Cys Leu Gly His Leu Ala Ala Ile Ile His Glu Ala Asn Glu
               740                 745                 750
   Glu Gln Gly Asn Ser Met Met Asn Leu Asp Trp Ser Trp Leu Thr Glu
           755                 760                 765
<210> 8
   <211> 2238
   <212> DNA
   <213> Artificial
<220>
   <223> IKKalpha hemmende Mutante
<400> 8
   atggagcggc ccccggggct gcggccgggc gcgggcgggc cctgggagat gcgggagcgg 60
   ctgggcaccg gcggcttcgg gaacgtctgt ctgtaccagc atcgggaact tgatctcaaa 120
   atagcaatta tgtcttgtcg cctagagcta agtaccaaaa acagagaacg atggtgccat 180
   gaaatccaga ttatgaagaa gttgaaccat gccaatgttg taaaggcctg tgatgttcct 240
   gaagaattga atattttgat tcatgatgtg cctcttctag caatggaata ctgttctgga 300
   ggagatctcc gaaagctgct caacaaacca gaaaattgtt gtggacttaa agaaagccag 360
   atactttctt tactaagtga tatagggtct gggattcgat atttgcatga aaacaaaatt 420
   atacatcgag atctaaaacc tgaaaacata gttcttcagg atgttggtgg aaagataata 480
   cataaaataa ttgatctggg atatgccaaa gatgttgatc aaggaagtct gtgtacatct 540
   tttgtgggaa cactgcagta tctggcccca gagctctttg agaataagcc ttacacagcc      600
   actgttgatt attggagctt tgggaccatg gtatttgaat gtattgctgg atataggcct      660
   tttttgcatc atctgcagcc atttacctgg catgagaaga ttaagaagaa ggatccaaag      720
   tgtatatttg catgtgaaga gatgtcacga gaagttcggt ttagtagcca tttacctcaa      780
   ccaaatagcc tttgtagttt aatagtagaa cccatggaaa actggctaca gttgatgttg      840
   aattgggacc ctcagcagag aggaggacct gttgacctta ctttgaagca gccaagatgt      900
   tttgtattaa tggatcacat tttgaatttg aagatagtac acatcctaaa tatgacttct      960
   gcaaagataa tttcttttct gttaccacct gatgaaagtc ttcattcatt acagtctcgt     1020
   attgagcgtg aaactggaat aaatactggt tctcaagaac ttctttcaga gacaggaatt     1080
   tctctggatc ctcggaaacc agcctctcaa tgtgttctag atggagttag aggctgtgat     1140
   agctatatgg tttatttgtt tgataaaagt aaaactgtat atgaagggcc atttgcttcc     1200
   agaagtttat ctgattgtgt aaattatatt gtacaggaca gcaaaataca gcttccaatt     1260
   atacagctgc gtaaagcgtg ggctgaagca gtgcactatg tgtctggact aaaagaagac     1320
   tatagcaggc tctttcaggg acaaagggca gcaatgttaa gtcttcttag atataatgct     1380
   aacttaacaa aaatgaagaa cactttgatc tcagcatcac aacaactgaa agctaaattg     1440
   gagttttttc acaaaagcat tcagcttgac ttggagagat acagcgagca gatgacgtat     1500
   gggatatctt cagaaaaaat gctaaaagca tggaaagaaa tggaagaaaa ggccatccac     1560
   tatgctgagg ttggtgtcat tggatacctg gaggatcaga ttatgtcttt gcatgctgaa     1620
   atcatggagc tacagaagag cccctatgga agacgtcagg gagacttgat ggaatctctg     1680
   gaacagcgtg ccattgatct atataagcag ttaaaacaca gaccttcaga tcactcctac     1740
   agtgacagca cagagatggt gaaaatcatt gtgcacactg tgcagagtca ggaccgtgtg     1800
   ctcaaggagc tgtttggtca tttgagcaag ttgttgggct gtaagcagaa gattattgat     1860
   ctactcccta aggtggaagt ggccctcagt aatatcaaag aagctgacaa tactgtcatg     1920
   ttcatgcagg gaaaaaggca gaaagaaatc tggcatctcc ttaaaattgc ctgtacacag     1980
   gccgctgccc gcgcccttgt gggagccgct ctggaaggtg cagtggcccc acaggccgcc     2040
   gcatggctgc cccctgctgc cgcagaacac gatcacgctc tggcctgtgt ggtggctcct     2100
   caagatgggg aggctgccgc acaaatgatc gaagaaaatt tgaactgcct tggccacttg     2160
   gccgctatta ttcacgaggc aaatgaggaa cagggcaata gtatgatgaa tcttgattgg     2220
   agttggttga cagaatga                                                   2238
<210> 9
   <211> 779
   <212> PRT
   <213> Artificial
<220>
   <223> IKKbeta hemmende Mutante
<400> 9
   Ser His His His His His Gly Asp Tyr Lys Asp Asp Asp Asp Lys Gly
   1               5                   10                  15
   Asp Ile Glu Gly Arg Gly His Met Ser Trp Ser Pro Ser Leu Thr Thr
               20                  25                  30
   Gln Thr Cys Gly Ala Trp Glu Met Lys Glu Arg Leu Gly Thr Gly Gly
           35                  40                  45
   Phe Gly Asn Val Ile Arg Trp His Asn Gln Glu Thr Gly Glu Gln Ile
       50                  55                  60
   Ala Ile Met Gln Cys Arg Gln Glu Leu Ser Pro Arg Asn Arg Glu Arg
   65                  70                  75                  80
   Trp Cys Leu Glu Ile Gln Ile Met Arg Arg Leu Thr His Pro Asn Val
                   85                  90                  95
   Val Ala Ala Arg Asp Val Pro Glu Gly Met Gln Asn Leu Ala Pro Asn
               100                 105                 110
   Asp Leu Pro Leu Leu Ala Met Glu Tyr Cys Gln Gly Gly Asp Leu Arg
           115                 120                 125
   Lys Tyr Leu Asn Gln Phe Glu Asn Cys Cys Gly Leu Arg Glu Gly Ala
       130                 135                 140
   Ile Leu Thr Leu Leu Ser Asp Ile Ala Ser Ala Leu Arg Tyr Leu His
   145                 150                 155                 160
   Glu Asn Arg Ile Ile His Arg Asp Leu Lys Pro Glu Asn Ile Val Leu
                   165                 170                 175
   Gln Gln Gly Glu Gln Arg Leu Ile His Lys Ile Ile Asp Leu Gly Tyr
               180                 185                 190
   Ala Lys Glu Leu Asp Gln Gly Ser Leu Cys Thr Ser Phe Val Gly Thr
           195                 200                 205
   Leu Gln Tyr Leu Ala Pro Glu Leu Leu Glu Gln Gln Lys Tyr Thr Val
       210                 215                 220
   Thr Val Asp Tyr Trp Ser Phe Gly Thr Leu Ala Phe Glu Cys Ile Thr
   225                 230                 235                 240
   Gly Phe Arg Pro Phe Leu Pro Asn Trp Gln Pro Val Gln Trp His Ser
                   245                 250                 255
   Lys Val Arg Gln Lys Ser Glu Val Asp Ile Val Val Ser Glu Asp Leu
               260                 265                 270
   Asn Gly Thr Val Lys Phe Ser Ser Ser Leu Pro Tyr Pro Asn Asn Leu
           275                 280                 285
   Asn Ser Val Leu Ala Glu Arg Leu Glu Lys Trp Leu Gln Leu Met Leu
       290                 295                 300
   Met Trp His Pro Arg Gln Arg Gly Thr Asp Pro Thr Tyr Gly Pro Asn
   305                 310                 315                 320
   Gly Cys Phe Lys Ala Leu Asp Asp Ile Leu Asn Leu Lys Leu Val His
                   325                 330                 335
   Ile Leu Asn Met Val Thr Gly Thr Ile His Thr Tyr Pro Val Thr Glu
               340                 345                 350
   Asp Glu Ser Leu Gln Ser Leu Lys Ala Arg Ile Gln Gln Asp Thr Gly
           355                 360                 365
   Ile Pro Glu Glu Asp Gln Glu Leu Leu Gln Glu Ala Gly Leu Ala Leu
       370                 375                 380
   Ile Pro Asp Lys Pro Ala Thr Gln Cys Ile Ser Asp Gly Lys Leu Asn
   385                 390                 395                 400
   Glu Gly His Thr Leu Asp Met Asp Leu Val Phe Leu Phe Asp Asn Ser
                   405                 410                 415
   Lys Ile Thr Tyr Glu Thr Gln Ile Ser Pro Arg Pro Gln Pro Glu Ser
               420                 425                 430
   Val Ser Cys Ile Leu Gln Glu Pro Lys Arg Asn Leu Ala Phe Phe Gln
           435                 440                 445
   Leu Arg Lys Val Trp Gly Gln Val Trp His Ser Ile Gln Thr Leu Lys
       450                 455                 460
   Glu Asp Cys Asn Arg Leu Gln Gln Gly Gln Arg Ala Ala Met Met Asn
   465                 470                 475                 480
   Leu Leu Arg Asn Asn Ser Cys Leu Ser Lys Met Lys Asn Ser Met Ala
                   485                 490                 495
   Ser Met Ser Gln Gln Leu Lys Ala Lys Leu Asp Phe Phe Lys Thr Ser
               500                 505                 510
   Ile Gln Ile Asp Leu Glu Lys Tyr Ser Glu Gln Thr Glu Phe Gly Ile
           515                 520                 525
   Thr Ser Asp Lys Leu Leu Leu Ala Trp Arg Glu Met Glu Gln Ala Val
       530                 535                 540
   Glu Leu Cys Gly Arg Glu Asn Glu Val Lys Leu Leu Val Glu Arg Met
   545 550 555 560
   Met Ala Leu Gln Thr Asp Ile Val Asp Leu Gln Arg Ser Pro Met Gly
   565 570 575
   Arg Lys Gln Gly Gly Thr Leu Asp Asp Leu Glu Glu Gln Ala Arg Glu
   580 585 590
   Leu Tyr Arg Arg Leu Arg Glu Lys Pro Arg Asp Gln Arg Thr Glu Gly
   595 600 605
   Asp Ser Gln Glu Met Val Arg Leu Leu Leu Gln Ala Ile Gln Ser Phe
   610 615 620
   Glu Lys Lys Val Arg Val Ile Tyr Thr Gln Leu Ser Lys Thr Val Val
   625 630 635 640
   Cys Lys Gln Lys Ala Leu Glu Leu Leu Pro Lys Val Glu Glu Val Val
   645 650 655
   Ser Leu Met Asn Glu Asp Glu Lys Thr Val Val Arg Leu Gln Glu Lys
   660 665 670
   Arg Gln Lys Glu Leu Trp Asn Leu Leu Lys Ile Ala Cys Ser Lys Val
   675 680 685
   Arg Gly Pro Val Ala Gly Ala Pro Asp Ala Met Asn Ala Ala Arg Leu
   690 695 700
   Ala Gln Pro Gly Gln Leu Met Ala Gln Pro Ala Thr Ala Ala Asn Ala
   705 710 715 720
   Leu Pro Glu Pro Ala Lys Lys Ala Glu Glu Leu Val Ala Glu Ala His
   725 730 735
   Asn Leu Cys Thr Leu Leu Glu Asn Ala Ile Gln Asp Thr Val Arg Glu
   740 745 750
   Gln Asp Gln Ser Phe Thr Ala Leu Asp Trp Ser Trp Leu Gln Thr Glu
   755 760 765
   Glu Glu Glu His Ser Cys Leu Glu Gln Ala Ser
   770 775
<210> 10
   <211> 2271
   <212> PRT
   <213> Artificial
<220>
   <223> IKKbeta hemmende Mutante
<400> 10
   Ala Thr Gly Ala Gly Cys Thr Gly Gly Thr Cys Ala Cys Cys Thr Thr
   1               5                   10                  15
   Cys Cys Cys Thr Gly Ala Cys Ala Ala Cys Gly Cys Ala Gly Ala Cys
               20                  25                  30
   Ala Thr Gly Cys Gly Gly Gly Gly Cys Cys Thr Gly Gly Gly Ala Ala
           35                  40                  45
   Ala Thr Gly Ala Ala Ala Gly Ala Gly Cys Gly Cys Cys Thr Thr Gly
       50                  55                  60
   Gly Gly Ala Cys Ala Gly Gly Gly Gly Gly Ala Thr Thr Thr Gly Gly
   65                  70                  75                  80
   Ala Ala Ala Thr Gly Thr Cys Ala Thr Cys Cys Gly Ala Thr Gly Gly
                   85                  90                  95
   Cys Ala Cys Ala Ala Thr Cys Ala Gly Gly Ala Ala Ala Cys Ala Gly
               100                 105                 110
   Gly Thr Gly Ala Gly Cys Ala Gly Ala Thr Thr Gly Cys Cys Ala Thr
           115                 120                 125
   Cys Ala Thr Gly Cys Ala Gly Thr Gly Cys Cys Gly Gly Cys Ala Gly
       130                 135                 140
   Gly Ala Gly Cys Thr Cys Ala Gly Cys Cys Cys Cys Cys Gly Gly Ala
   145                 150                 155                 160
   Ala Cys Cys Gly Ala Gly Ala Gly Cys Gly Gly Thr Gly Gly Thr Gly
                   165                 170                 175
   Cys Cys Thr Gly Gly Ala Gly Ala Thr Cys Cys Ala Gly Ala Thr Cys
               180                 185                 190
   Ala Thr Gly Ala Gly Ala Ala Gly Gly Cys Thr Gly Ala Cys Cys Cys
           195                 200                 205
   Ala Cys Cys Cys Cys Ala Ala Thr Gly Thr Gly Gly Thr Gly Gly Cys
       210                 215                 220
   Thr Gly Cys Cys Cys Gly Ala Gly Ala Thr Gly Thr Cys Cys Cys Thr
   225                 230                 235                 240
   Gly Ala Gly Gly Gly Gly Ala Thr Gly Cys Ala Gly Ala Ala Cys Thr
                   245                 250                 255
   Thr Gly Gly Cys Gly Cys Cys Cys Ala Ala Thr Gly Ala Cys Cys Thr
   260 265 270
   Gly Cys Cys Cys Cys Thr Gly Cys Thr Gly Gly Cys Cys Ala Thr Gly
   275 280 285
   Gly Ala Gly Thr Ala Cys Thr Gly Cys Cys Ala Ala Gly Gly Ala Gly
   290 295 300
   Gly Ala Gly Ala Thr Cys Thr Cys Cys Gly Gly Ala Ala Gly Thr Ala
   305 310 315 320
   Cys Cys Thr Gly Ala Ala Cys Cys Ala Gly Thr Thr Thr Gly Ala Gly
   325 330 335
   Ala Ala Cys Thr Gly Cys Thr Gly Thr Gly Gly Thr Cys Thr Gly Cys
   340 345 350
   Gly Gly Gly Ala Ala Gly Gly Thr Gly Cys Cys Ala Thr Cys Cys Thr
   355 360 365
   Cys Ala Cys Cys Thr Thr Gly Cys Thr Gly Ala Gly Thr Gly Ala Cys
   370 375 380
   Ala Thr Thr Gly Cys Cys Thr Cys Thr Gly Cys Gly Cys Thr Thr Ala
   385 390 395 400
   Gly Ala Thr Ala Cys Cys Thr Thr Cys Ala Thr Gly Ala Ala Ala Ala
   405 410 415
   Cys Ala Gly Ala Ala Thr Cys Ala Thr Cys Cys Ala Thr Cys Gly Gly
   420 425 430
   Gly Ala Thr Cys Thr Ala Ala Ala Gly Cys Cys Ala Gly Ala Ala Ala
   435 440 445
   Ala Cys Ala Thr Cys Gly Thr Cys Cys Thr Gly Cys Ala Gly Cys Ala
   450 455 460
   Ala Gly Gly Ala Gly Ala Ala Cys Ala Gly Ala Gly Gly Thr Thr Ala
   465 470 475 480
   Ala Thr Ala Cys Ala Cys Ala Ala Ala Ala Thr Thr Ala Thr Thr Gly
   485 490 495
   Ala Cys Cys Thr Ala Gly Gly Ala Thr Ala Thr Gly Cys Cys Ala Ala
   500 505 510
   Gly Gly Ala Gly Cys Thr Gly Gly Ala Thr Cys Ala Gly Gly Gly Cys
   515 520 525
   Ala Gly Thr Cys Thr Thr Thr Gly Cys Ala Cys Ala Thr Cys Ala Thr
   530 535 540
   Thr Cys Gly Thr Gly Gly Gly Gly Ala Cys Cys Cys Thr Gly Cys Ala
   545 550 555 560
   Gly Thr Ala Cys Cys Thr Gly Gly Cys Cys Cys Cys Ala Gly Ala Gly
   565 570 575
   Cys Thr Ala Cys Thr Gly Gly Ala Gly Cys Ala Gly Cys Ala Gly Ala
   580 585 590
   Ala Gly Thr Ala Cys Ala Cys Ala Gly Thr Gly Ala Cys Cys Gly Thr
   595 600 605
   Cys Gly Ala Cys Thr Ala Cys Thr Gly Gly Ala Gly Cys Thr Thr Cys
   610 615 620
   Gly Gly Cys Ala Cys Cys Cys Thr Gly Gly Cys Cys Thr Thr Thr Gly
   625 630 635 640
   Ala Gly Thr Gly Cys Ala Thr Cys Ala Cys Gly Gly Gly Cys Thr Thr
   645 650 655
   Cys Cys Gly Gly Cys Cys Cys Thr Thr Cys Cys Thr Cys Cys Cys Cys
   660 665 670
   Ala Ala Cys Thr Gly Gly Cys Ala Gly Cys Cys Cys Gly Thr Gly Cys
   675 680 685
   Ala Gly Thr Gly Gly Cys Ala Thr Thr Cys Ala Ala Ala Ala Gly Thr
   690 695 700
   Gly Cys Gly Gly Cys Ala Gly Ala Ala Gly Ala Gly Thr Gly Ala Gly
   705 710 715 720
   Gly Thr Gly Gly Ala Cys Ala Thr Thr Gly Thr Thr Gly Thr Thr Ala
   725 730 735
   Gly Cys Gly Ala Ala Gly Ala Cys Thr Thr Gly Ala Ala Thr Gly Gly
   740 745 750
   Ala Ala Cys Gly Gly Thr Gly Ala Ala Gly Thr Thr Thr Thr Cys Ala
   755 760 765
   Ala Gly Cys Thr Cys Thr Thr Thr Ala Cys Cys Cys Thr Ala Cys Cys
   770 775 780
   Cys Cys Ala Ala Thr Ala Ala Thr Cys Thr Thr Ala Ala Cys Ala Gly
   785 790 795 800
   Thr Gly Thr Cys Cys Thr Gly Gly Cys Thr Gly Ala Gly Cys Gly Ala
   805 810 815
   Cys Thr Gly Gly Ala Gly Ala Ala Gly Thr Gly Gly Cys Thr Gly Cys
   820 825 830
   Ala Ala Cys Thr Gly Ala Thr Gly Cys Thr Gly Ala Thr Gly Thr Gly
   835 840 845
   Gly Cys Ala Cys Cys Cys Cys Cys Gly Ala Cys Ala Gly Ala Gly Gly
   850 855 860
   Gly Gly Cys Ala Cys Gly Gly Ala Thr Cys Cys Cys Ala Cys Gly Thr
   865 870 875 880
   Ala Thr Gly Gly Gly Cys Cys Cys Ala Ala Thr Gly Gly Cys Thr Gly
   885 890 895
   Cys Thr Thr Cys Ala Ala Gly Gly Cys Cys Cys Thr Gly Gly Ala Thr
   900 905 910
   Gly Ala Cys Ala Thr Cys Thr Thr Ala Ala Ala Cys Thr Thr Ala Ala
   915 920 925
   Ala Gly Cys Thr Gly Gly Thr Thr Cys Ala Thr Ala Thr Cys Thr Thr
   930 935 940
   Gly Ala Ala Cys Ala Thr Gly Gly Thr Cys Ala Cys Gly Gly Gly Cys
   945 950 955 960
   Ala Cys Cys Ala Thr Cys Cys Ala Cys Ala Cys Cys Thr Ala Cys Cys
   965 970 975
   Cys Thr Gly Thr Gly Ala Cys Ala Gly Ala Gly Gly Ala Thr Gly Ala
   980 985 990
   Gly Ala Gly Thr Cys Thr Gly Cys Ala Gly Ala Gly Cys Thr Thr Gly
   995 1000 1005
   Ala Ala Gly Gly Cys Cys Ala Gly Ala Ala Thr Cys Cys Ala Ala
   1010 1015 1020
   Cys Ala Gly Gly Ala Cys Ala Cys Gly Gly Gly Cys Ala Thr Cys
   1025 1030 1035
   Cys Cys Ala Gly Ala Gly Gly Ala Gly Gly Ala Cys Cys Ala Gly
   1040 1045 1050
   Gly Ala Gly Cys Thr Gly Cys Thr Gly Cys Ala Gly Gly Ala Ala
   1055 1060 1065
   Gly Cys Gly Gly Gly Cys Cys Thr Gly Gly Cys Gly Thr Thr Gly
   1070 1075 1080
   Ala Thr Cys Cys Cys Cys Gly Ala Thr Ala Ala Gly Cys Cys Thr
   1085 1090 1095
   Gly Cys Cys Ala Cys Thr Cys Ala Gly Thr Gly Thr Ala Thr Thr
   1100 1105 1110
   Thr Cys Ala Gly Ala Cys Gly Gly Cys Ala Ala Gly Thr Thr Ala
   1115 1120 1125
   Ala Ala Thr Gly Ala Gly Gly Gly Cys Cys Ala Cys Ala Cys Ala
   1130 1135 1140
   Thr Thr Gly Gly Ala Cys Ala Thr Gly Gly Ala Thr Cys Thr Thr
   1145 1150 1155
   Gly Thr Thr Thr Thr Thr Cys Thr Cys Thr Thr Thr Gly Ala Cys
   1160 1165 1170
   Ala Ala Cys Ala Gly Thr Ala Ala Ala Ala Thr Cys Ala Cys Cys
   1175 1180 1185
   Thr Ala Thr Gly Ala Gly Ala Cys Thr Cys Ala Gly Ala Thr Cys
   1190 1195 1200
   Thr Cys Cys Cys Cys Ala Cys Gly Gly Cys Cys Cys Cys Ala Ala
   1205 1210 1215
   Cys Cys Thr Gly Ala Ala Ala Gly Thr Gly Thr Cys Ala Gly Cys
   1220 1225 1230
   Thr Gly Thr Ala Thr Cys Cys Thr Thr Cys Ala Ala Gly Ala Gly
   1235 1240 1245
   Cys Cys Cys Ala Ala Gly Ala Gly Gly Ala Ala Thr Cys Thr Cys
   1250 1255 1260
   Gly Cys Cys Thr Thr Cys Thr Thr Cys Cys Ala Gly Cys Thr Gly
   1265 1270 1275
   Ala Gly Gly Ala Ala Gly Gly Thr Gly Thr Gly Gly Gly Gly Cys
   1280 1285 1290
   Cys Ala Gly Gly Thr Cys Thr Gly Gly Cys Ala Cys Ala Gly Cys
   1295 1300 1305
   Ala Thr Cys Cys Ala Gly Ala Cys Cys Cys Thr Gly Ala Ala Gly
   1310 1315 1320
   Gly Ala Ala Gly Ala Thr Thr Gly Cys Ala Ala Cys Cys Gly Gly
   1325 1330 1335
   Cys Thr Gly Cys Ala Gly Cys Ala Gly Gly Gly Ala Cys Ala Gly
   1340 1345 1350
   Cys Gly Ala Gly Cys Cys Gly Cys Cys Ala Thr Gly Ala Thr Gly
   1355 1360 1365
   Ala Ala Thr Cys Thr Cys Cys Thr Cys Cys Gly Ala Ala Ala Cys
   1370 1375 1380
   Ala Ala Cys Ala Gly Cys Thr Gly Cys Cys Thr Cys Thr Cys Cys
   1385 1390 1395
   Ala Ala Ala Ala Thr Gly Ala Ala Gly Ala Ala Thr Thr Cys Cys
   1400 1405 1410
   Ala Thr Gly Gly Cys Thr Thr Cys Cys Ala Thr Gly Thr Cys Thr
   1415 1420 1425
   Cys Ala Gly Cys Ala Gly Cys Thr Cys Ala Ala Gly Gly Cys Cys
   1430 1435 1440
   Ala Ala Gly Thr Thr Gly Gly Ala Thr Thr Thr Cys Thr Thr Cys
   1445 1450 1455
   Ala Ala Ala Ala Cys Cys Ala Gly Cys Ala Thr Cys Cys Ala Gly
   1460 1465 1470
   Ala Thr Thr Gly Ala Cys Cys Thr Gly Gly Ala Gly Ala Ala Gly
   1475 1480 1485
   Thr Ala Cys Ala Gly Cys Gly Ala Gly Cys Ala Ala Ala Cys Cys
   1490 1495 1500
   Gly Ala Gly Thr Thr Thr Gly Gly Gly Ala Thr Cys Ala Cys Ala
   1505 1510 1515
   Thr Cys Ala Gly Ala Thr Ala Ala Ala Cys Thr Gly Cys Thr Gly
   1520 1525 1530
   Cys Thr Gly Gly Cys Cys Thr Gly Gly Ala Gly Gly Gly Ala Ala
   1535 1540 1545
   Ala Thr Gly Gly Ala Gly Cys Ala Gly Gly Cys Thr Gly Thr Gly
   1550 1555 1560
   Gly Ala Gly Cys Thr Cys Thr Gly Thr Gly Gly Gly Cys Gly Gly
   1565 1570 1575
   Gly Ala Gly Ala Ala Cys Gly Ala Ala Gly Thr Gly Ala Ala Ala
   1580 1585 1590
   Cys Thr Cys Cys Thr Gly Gly Thr Ala Gly Ala Ala Cys Gly Gly
   1595 1600 1605
   Ala Thr Gly Ala Thr Gly Gly Cys Thr Cys Thr Gly Cys Ala Gly
   1610 1615 1620
   Ala Cys Cys Gly Ala Cys Ala Thr Thr Gly Thr Gly Gly Ala Cys
   1625 1630 1635
   Thr Thr Ala Cys Ala Gly Ala Gly Gly Ala Gly Cys Cys Cys Cys
   1640 1645 1650
   Ala Thr Gly Gly Gly Cys Cys Gly Gly Ala Ala Gly Cys Ala Gly
   1655 1660 1665
   Gly Gly Gly Gly Gly Ala Ala Cys Gly Cys Thr Gly Gly Ala Cys
   1670 1675 1680
   Gly Ala Cys Cys Thr Ala Gly Ala Gly Gly Ala Gly Cys Ala Ala
   1685 1690 1695
   Gly Cys Ala Ala Gly Gly Gly Ala Gly Cys Thr Gly Thr Ala Cys
   1700 1705 1710
   Ala Gly Gly Ala Gly Ala Cys Thr Ala Ala Gly Gly Gly Ala Ala
   1715 1720 1725
   Ala Ala Ala Cys Cys Thr Cys Gly Ala Gly Ala Cys Cys Ala Gly
   1730 1735 1740
   Cys Gly Ala Ala Cys Thr Gly Ala Gly Gly Gly Thr Gly Ala Cys
   1745 1750 1755
   Ala Gly Thr Cys Ala Gly Gly Ala Ala Ala Thr Gly Gly Thr Ala
   1760 1765 1770
   Cys Gly Gly Cys Thr Gly Cys Thr Gly Cys Thr Thr Cys Ala Gly
   1775 1780 1785
   Gly Cys Ala Ala Thr Thr Cys Ala Gly Ala Gly Cys Thr Thr Cys
   1790 1795 1800
   Gly Ala Gly Ala Ala Gly Ala Ala Ala Gly Thr Gly Cys Gly Ala
   1805 1810 1815
   Gly Thr Gly Ala Thr Cys Thr Ala Thr Ala Cys Gly Cys Ala Gly
   1820 1825 1830
   Cys Thr Cys Ala Gly Thr Ala Ala Ala Ala Cys Thr Gly Thr Gly
   1835 1840 1845
   Gly Thr Thr Thr Gly Cys Ala Ala Gly Cys Ala Gly Ala Ala Gly
   1850 1855 1860
   Gly Cys Gly Cys Thr Gly Gly Ala Ala Cys Thr Gly Thr Thr Gly
   1865 1870 1875
   Cys Cys Cys Ala Ala Gly Gly Thr Gly Gly Ala Ala Gly Ala Gly
   1880 1885 1890
   Gly Thr Gly Gly Thr Gly Ala Gly Cys Thr Thr Ala Ala Thr Gly
   1895 1900 1905
   Ala Ala Thr Gly Ala Gly Gly Ala Thr Gly Ala Gly Ala Ala Gly
   1910 1915 1920
   Ala Cys Thr Gly Thr Thr Gly Thr Cys Cys Gly Gly Cys Thr Gly
   1925 1930 1935
   Cys Ala Gly Gly Ala Gly Ala Ala Gly Cys Gly Gly Cys Ala Gly
   1940 1945 1950
   Ala Ala Gly Gly Ala Gly Cys Thr Cys Thr Gly Gly Ala Ala Thr
   1955 1960 1965
   Cys Thr Cys Cys Thr Gly Ala Ala Gly Ala Thr Thr Gly Cys Thr
   1970 1975 1980
   Thr Gly Thr Ala Gly Cys Ala Ala Gly Gly Thr Cys Cys Gly Thr
   1985 1990 1995
   Gly Gly Thr Cys Cys Thr Gly Thr Cys Gly Cys Thr Gly Gly Ala
   2000 2005 2010
   Gly Cys Cys Cys Cys Gly Gly Ala Thr Gly Cys Cys Ala Thr Gly
   2015 2020 2025
   Ala Ala Thr Gly Cys Cys Gly Cys Thr Cys Gly Ala Cys Thr Thr
   2030 2035 2040
   Gly Cys Cys Cys Ala Gly Cys Cys Thr Gly Gly Gly Cys Ala Gly
   2045 2050 2055
   Cys Thr Gly Ala Thr Gly Gly Cys Thr Cys Ala Gly Cys Cys Cys
   2060 2065 2070
   Gly Cys Cys Ala Cys Gly Gly Cys Cys Gly Cys Cys Ala Ala Cys
   2075 2080 2085
   Gly Cys Cys Thr Thr Ala Cys Cys Thr Gly Ala Gly Cys Cys Ala
   2090 2095 2100
   Gly Cys Cys Ala Ala Gly Ala Ala Gly Gly Cys Thr Gly Ala Ala
   2105 2110 2115
   Gly Ala Ala Cys Thr Gly Gly Thr Gly Gly Cys Thr Gly Ala Ala
   2120 2125 2130
   Gly Cys Ala Cys Ala Thr Ala Ala Cys Cys Thr Cys Thr Gly Cys
   2135 2140 2145
   Ala Cys Cys Cys Thr Gly Cys Thr Ala Gly Ala Ala Ala Ala Thr
   2150 2155 2160
   Gly Cys Cys Ala Thr Ala Cys Ala Gly Gly Ala Cys Ala Cys Thr
   2165 2170 2175
   Gly Thr Gly Ala Gly Gly Gly Ala Ala Cys Ala Ala Gly Ala Cys
   2180 2185 2190
   Cys Ala Gly Ala Gly Thr Thr Thr Cys Ala Cys Gly Gly Cys Cys
   2195 2200 2205
   Cys Thr Ala Gly Ala Cys Thr Gly Gly Ala Gly Cys Thr Gly Gly
   2210 2215 2220
   Thr Thr Ala Cys Ala Gly Ala Cys Gly Gly Ala Ala Gly Ala Ala
   2225 2230 2235
   Gly Ala Ala Gly Ala Gly Cys Ala Cys Ala Gly Cys Thr Gly Cys
   2240 2245 2250
   Cys Thr Gly Gly Ala Gly Cys Ala Gly Gly Cys Cys Thr Cys Ala
   2255 2260 2265
   Thr Gly Ala
   2270

## Beschreibung

Die Erfindung betrifft die in den Ansprüchen charakterisierten Ausführungsformen, nämlich dendritische Zellen, die durch RNA-Transfektion in ihrem NFκB-Signalweg manipuliert sind, deren Herstellung und Verwendung.

### Hintergrund der Erfindung

In der Beschreibung werden eine Reihe an Dokumenten inklusive Patentanmeldungen und Hersteller-Gebrauchsanweisungen aus dem Stand der Technik genannt. Der Offenbarungsgehalt dieser Dokumente wird als nicht relevant für die Patentfähigkeit der Erfindung angesehen.

Dendritische Zellen (DZ) stellen das Bindeglied zwischen der angeborenen und der adaptiven Immunantwort dar. Sie sind in der Lage, Pathogene als solche zu erkennen und eine adaptive (also eine auf das spezielle Pathogen zugeschnittene) Immunantwort zu initialisieren und zu dirigieren. Auch sind DZ fähig, in Abwesenheit von Pathogenen Toleranz gegen körpereigene Antigene zu vermitteln. Somit stellen DZ den Schlüssel zur gezielten Induktion von Immunantworten, aber auch zur Vermittlung von immunologischer Toleranz dar. Das Zytokin IL-12p70 spielt eine wichtige Rolle bei der Induktion von zellvermittelter Immunität, während das Zytokin IL-10 an der Induktion von humoraler Immunität aber auch von Toleranz beteiligt ist. Die Immuntherapie maligner Erkrankungen unter Verwendung von DZ als Adjuvans fand bereits in diversen klinischen Studien Anwendung, wobei die Sicherheit und Machbarkeit dieser Methode gezeigt werden konnte. Allerdings blieben die klinischen Erfolge hinter den Erwartungen zurück, obwohl regelmäßig Immunantworten der Patienten gegen die verwendeten Antigene *in vitro* nachweisbar waren. Durch die Erzeugung der DZ in Zellkultur besteht die Möglichkeit, diese gezielt zu manipulieren. Hierbei ist es von Vorteil, DZ zu erzeugen, die in der Lage sind, langlebige Gedächtnis-T-Zellen zu induzieren und sich dabei resistent gegenüber regulatorischen T-Zellen (Treg) und anderen tolerogenen Mechanismen zu verhalten. Langlebige Gedächtnis-T-Zellen können bei erneutem Kontakt mit einem Antigen eine schnellere und effizientere Sekundärantwort vermitteln. Diese Erinnerungsfunktion kann durch CD4-positive und durch CD8-positive T-Gedächtniszellen übernommen werden. Langlebige Gedächtnis-T-Zellen stehen im Gegensatz zu Effektorzellen, die nur eine kurze Lebenszeit haben und meist nach einer Immunantwort durch Aktivierungsinduzierten Zelltod (AICD, *activation-inducing cell death)* sterben. Zwischen den beiden Zelltypen gibt es jedoch Übergangsformen, wie die Effektor-Gedächtnis-Zellen. Diese sind wie die Effektorzellen in der Lage im ganzen Körper zu patrouillieren, und bei Antigenkontakt Effektorfunktion ausüben, sie können sich auch noch vermehren und sind langlebiger als die Effektorzellen. Andererseits ist auch der Einsatz von DZ zur gezielten Behandlung von Autoimmunität und Allergie denkbar, da DZ unter bestimmten Bedingungen Immunantworten unterdrücken und Toleranz vermitteln können. Verbesserte Methoden und Protokolle zur Herstellung der verschiedenen Arten von DZ sind deswegen von großem Interesse und werden weltweit erforscht. Eine Übersicht des gegenwärtigen Standes der Technik wird durch Boczkowski und Nair, Expert Opin. Biol. Ther. 10 (4) (2010), 563-574 sowie durch Kaisho und Tanaka, Trends in Immunology 29 (7) (2008), 329-336 vermittelt.
DZ verfügen über eine Vielzahl an Oberflächenrezeptoren, mit denen sie verschiedenste Pathogene erkennen können. Zusätzlich sind DZ in der Lage, diverse körpereigene Botenstoffe wie Zytokine und Chemokine, sowie Oberflächenmoleküle auf anderen Zellen des Immunsystems wahrzunehmen. Über intrazelluläre Signalwege verrechnet die DZ die diversen eingehenden Signale, wodurch verschiedene Differenzierungsprogramme ausgelöst werden. Die gezielte Manipulation dieser Signalwege kann die Erzeugung von maßgeschneiderten DZ ermöglichen, die dadurch besser geeignet sind entweder Immunität (in der Krebs-Immuntherapie) oder Toleranz (in der Behandlung von Autoimmunerkrankungen und Allergien) zu vermitteln.
Verschiedene Möglichkeiten, durch genetische Manipulation in die Signalwege in der DZ einzugreifen, wurden bereits formuliert und durchgeführt. Dabei treten jedoch verschiedene Hemmnisse auf, vor allem bei der Manipulation von menschlichen DZ. Die genetische Manipulation im Rahmen einer Therapie stellt sich als bedenklich dar, und die somatische Gentherapie ist streng reguliert. Außerdem sind die Möglichkeiten, die am meisten in der Medizin verwendeten menschlichen DZ (die Monozyten-abgeleiteten DZ), genetisch zu verändern sehr begrenzt, und nur die Verwendung von viralen Transfektionssystemen, die aus Lentiviren oder Adenoviren entwickelt wurden, führt bisher überhaupt zum Erfolg. So offenbart US-A1 2009/0202492 eine konstitutiv-aktive Form von IKKα und/oder IKKβ erhalten durch Substitution der Serinreste an den Positionen 176 und 180 für IKKα und 177 und 181 für IKKβ, die mittels eines Adenovirus exprimiert wird. Die Verwendung solcher Vehikel zum Einbringen von DNA wird jedoch sehr kritisch betrachtet, und bringt weitere Risiken mit sich. So werden durch Lentiviren immer auch virale Sequenzen in das Genom der Zelle eingebaut. Dadurch können aktive zelleigene Gene zerstört werden oder die viralen Promotoren können Gene aktivieren, die sonst inaktiv wären. Da die Integration in das Genom jedoch zufällig erfolgt, ist nicht vorhersehbar, welche Gene betroffen sein können. Falls es sich um Tumorsuppressorgene oder Onkogene handelt, die zerstört bzw. aktiviert werden, kann die Zelle, im schlimmsten Fall, selbst zur Tumorzelle werden. Auch kann sich die induzierte Immunantwort statt gegen die gewünschten Antigene gegen die viralen Produkte richten. Letzteres gilt auch für adenovirale Systeme, wobei hier die Immunantwort sehr heftig ausfallen kann, da viele Menschen bereits eine bestehende Immunantwort gegen Adenoviren haben. Eine derartige heftige Immunreaktion gegen einen adenoviralen Vektor führte im Jahr 1999 sogar zu einem Todesfall.
Ein zentraler Signalweg der DZ ist die NFκB-Signalkaskade. Die Stimulierung vieler der Oberflächenrezeptoren der DZ führt zu einer Aktivierung dieser Kaskade, wobei inhibitorische Proteine durch Phosphorylierung destabilisiert werden, so dass Transkriptionsfaktoren in den Kern gelangen und dort die Transkription diverser Gene verursachen. Die Kinasen, die diese Phosphorylierung durchführen, werden als IKK (Inhibitor-von-kappa-Kinasen) bezeichnet.

### Kurzbeschreibung der Erfindung

Es wurde nunmehr gefunden, dass dendritische Zellen (nachfolgend kurz "DZ") durch RNA-Transfektion und Expression von mutierten signalübertragenden Proteinen des NFκB-Signalweges in ihrem NFκB-Signalweg manipuliert werden können. Es konnten sowohl konstitutiv aktive als auch dominant negative Mutanten gefunden werden. Durch die Reifung der DZ vor oder nach der RNA-Transfektion können DZ mit verschiedenen Phänotypen und Zytokinprofilen erzeugt werden, wobei die Zytokine IL-12p70 (zur Induktion von Immunität) und IL-10 (zur Induktion von Toleranz bzw. zur Erzeugung immunsupprimierter Phänotypen) zentrale Rollen spielen. Die Erfindung betrifft somit wie in den Ansprüchen ausgeführt:
(1) Dendritische Zellen (DZ), die durch RNA-Transfektion mit einer oder mehreren Nukleotidsequenzen, die für ein mutiertes signalübertragendes Protein des NFκB-Signalweges kodieren, in ihrem NFκB-Signalweg manipuliert sind;
(2) ein Verfahren zur Herstellung von NFκB-Signalweg manipulierten DZ nach (1), umfassend die RNA-Transfektion von unreifen oder reifen DZ mit einer oder mehreren Nukleotidsequenzen, die für ein mutiertes signalübertragendes Protein des NFκB-Signalweges kodieren;
(3) eine Zusammensetzung, pharmazeutische Zusammensetzung oder ein Medikament umfassend DZ nach (1);
(4) die Verwendung der DZ nach (1) zur Stimulation autologer CD8⁺ T-Zellen ex *vivo*;
(5) die Verwendung der DZ nach (1) zur Herstellung eines Medikaments zur Behandlung und Prävention von Krebs, Infektionskrankheiten wie HIV vermittelte AIDS oder Autoimmunerkrankungen in einem Patienten; und gleichermaßen die DZ nach (1) zur Verwendung bei der Behandlung und Prävention von Krebs, Infektionskrankheiten oder Autoimmunerkrankungen in einem Patienten
(6) ein Verfahren zur Expansion von T Zellen, einschließlich der Stimulation autologer CD8⁺ T-Zellen *ex vivo,* umfassend Stimulieren der Zellen mit DZ nach (1).
(7) Weiterhin ist in der Beschreibung ein Verfahren zur Behandlung von Krebs, Infektionskrankheiten oder Autoimmunerkrankungen in einem Patienten, umfassend das Verabreichen der DZ nach (1) an den Patienten beschrieben.

### Kurzbeschreibung der Figuren

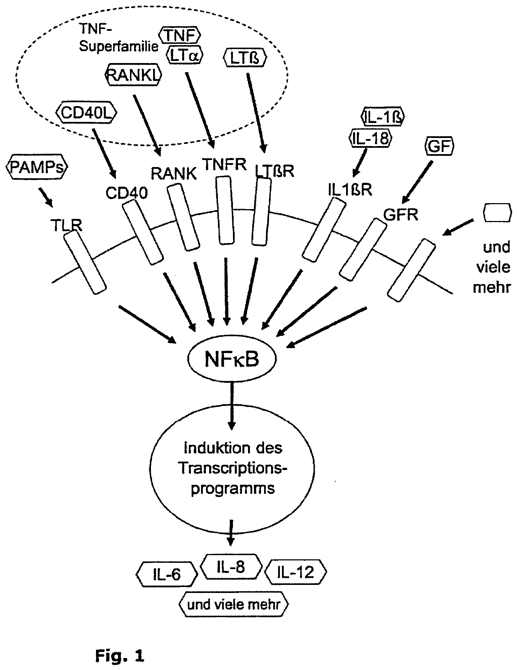

Fig. 1: NFκB steht im Mittelpunkt der DZ Reifungssignalkette. Eine Vielzahl von Oberflächenrezeptoren, die von Gefahrsignalen ausgelöst werden, und von proinflammatorischen Stimulantien, von denen bekannt ist, dass sie die DZ Reifung auslösen, verursachen die Aktivierung von NFκB. Die NFκB Aktivierung wiederum verursacht die Ausschüttung von wichtigen Zytokinen wie IL-12p70 und phänotypische Veränderungen der DZ.

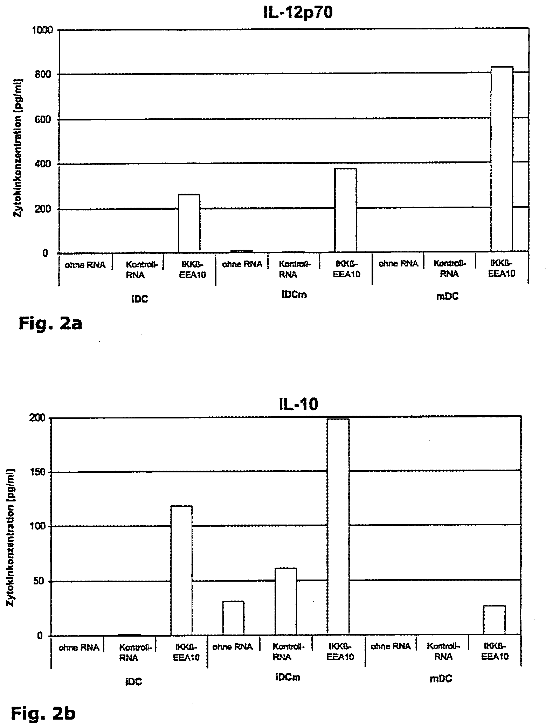

Fig. 2: Sekretion von IL-12p70 (a) und IL-10 (b) durch IKKβ-EEA10-RNA-elektroporierte dendritische Zellen.

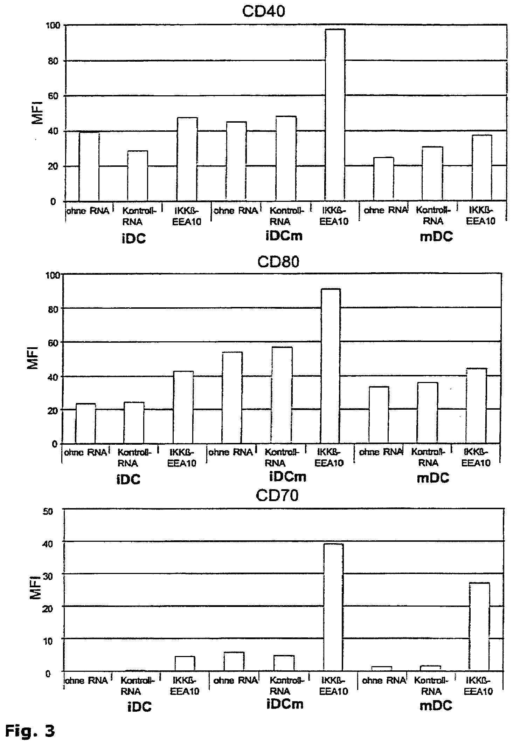

Fig. 3: Expression von Oberflächenmarkern auf dendritischen Zellen, transfiziert mit der NFκB-Signalweg-Komponente IKKβ-EEA10.

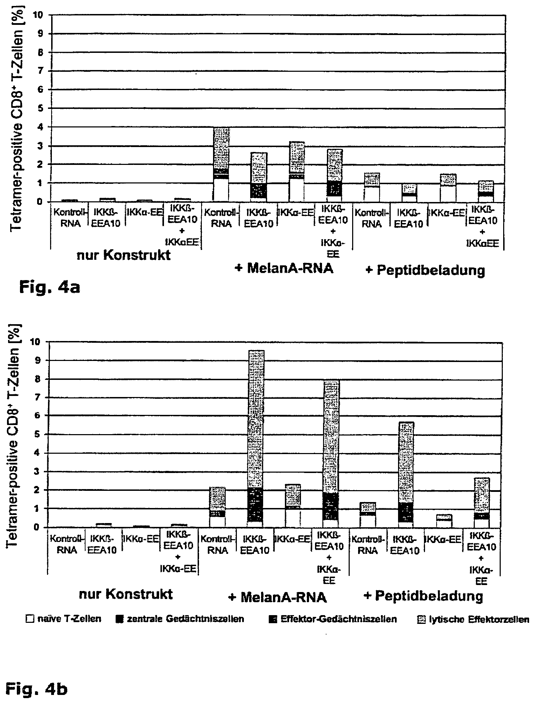

Fig. 4: Tetramerfärbung der Stimulation autologer T-Zellen mit dendritischen Zellen, elektroporiert mit einer oder zwei RNAs, die konstitutiv aktivierte Mutanten von IKKα und IKKβ kodieren und analysiert nach einem Priming (a) und nach einer Restimulation (b).

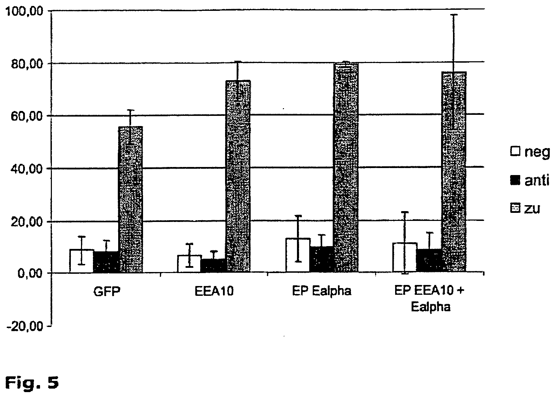

Fig. 5: Migration reifer dendritischer Zellen 24 h nach RNA-Transfektion mit einer oder zwei RNAs, die konstitutiv aktivierte Mutanten von IKKα und IKKβ kodieren.

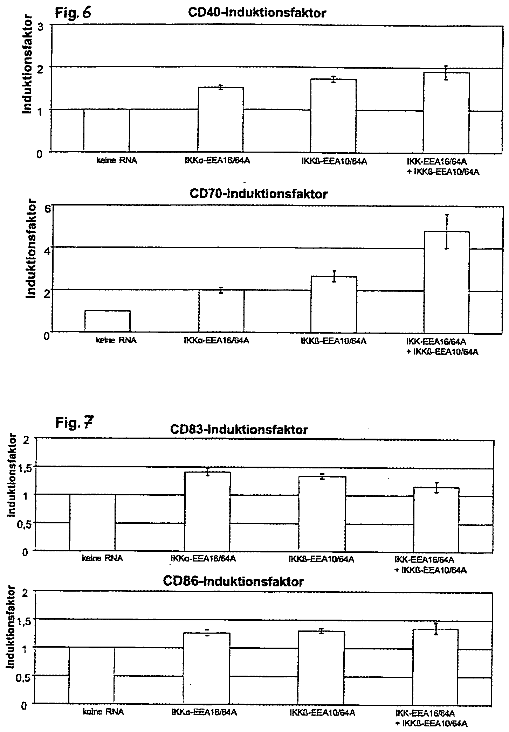

Fig.6: Induktionsfaktor von Oberflächenmarkern auf DZ, die mit Komponenten des NFκB-Signalwegs elektroporiert wurden. DZ wurden aus Monocyten während einer sechstägigen Kultur mit GM-CSF und IL-4 erzeugt. Am Tag 6 sind die DZ 24h lang nach Zugabe eines Standard-Reifungscocktail (IL-1β, IL-6, TNFα und PGE2) gereift (mDZ). Dann wurden die DZ ohne RNA mit IKKα-EE-A16-RNA (aktiviert alternativen Signalweg), IKKβ-EEA10-RNA (aktiviert klassischen Signalweg) allein oder in Kombination (jeweils 15 µg RNA) elektroporiert. Diese DZ wurden 24h nach der EP mit Antikörpern gegen CD 40 und CD70 angefärbt und durch FACS analysiert. Der Mittelwert von 8 unabhängigen Spendern ist mit dem Standardfehler des Mittelwerts angegeben.
Fig.7: Induktionsfaktor von Oberflächenmarkern auf DZ, die mit Komponenten des NFκB-Signalwegs elektroporiert wurden. DZ wurden aus Monocyten während einer sechstägigen Kultur mit GM-CSF und IL-4 erzeugt. Am Tag 6 sind die DZ 24h lang nach Zugabe eines Standard-Reifungscocktail (IL-1β, IL-6, TNFα und PGE2) gereift (mDZ). Dann wurden die DZ ohne RNA, mit IKKα-EE-A16-RNA (aktiviert alternativen Signalweg), IKKβ-EEA10-RNA (aktiviert klassischen Signalweg) allein oder in Kombination (jeweils 15 µg RNA) elektroporiert. Diese DZ wurden 24h nach der EP mit Antikörpern gegen CD83 und CD86 angefärbt und durch FACS analysiert. Der Mittelwert von 8 unabhängigen Spendern ist mit dem Standardfehler des Mittelwerts angegeben.

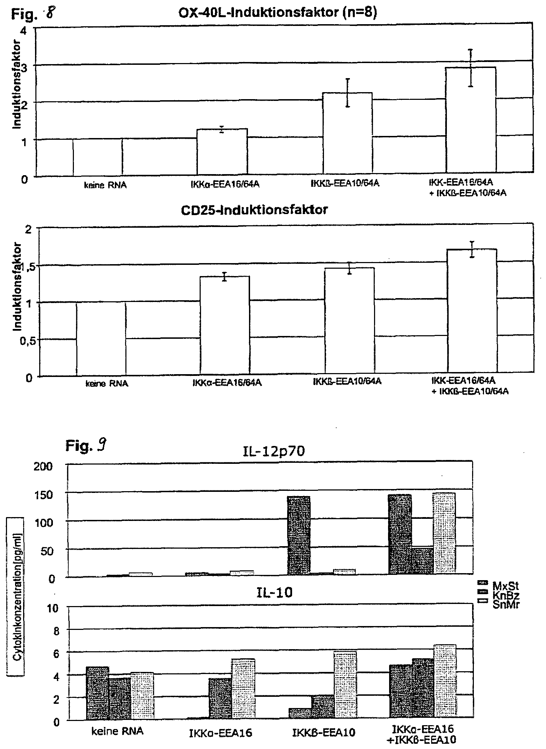

Fig.8: Induktionsfaktor von Oberflächenmarkern auf DZ, die mit Komponenten des NFκB-Signalwegs elektroporiert wurden. DZ wurden aus Monocyten während einer sechstägigen Kultur mit GM-CSF und IL-4 erzeugt. Am Tag 6 sind die DZ 24h lang nach Zugabe eines Standard-Reifungscocktail (IL-1β, IL-6, TNFα und PGE2) gereift (mDZ). Dann wurden die DZ ohne RNA, mit IKKα-EE-A16-RNA (aktiviert alternativen Signalweg), IKKβ-EEA10-RNA (aktiviert klassischen Signalweg) allein oder in Kombination (jeweils 15 µg RNA) elektroporiert. Diese DZ wurden 24h nach der EP mit Antikörpern gegen OX-40L und CD25 angefärbt und durch FACS analysiert. Der Mittelwert von 8 unabhängigen Spendern ist mit dem Standardfehler des Mittelwerts angegeben.
Fig.9: Sekretion der Cytokine IL-12p70 und IL-10 von DZ, die mit Komponenten des NFκB-Signalwegs elektroporiert wurden. DZ wurden aus Monocyten während einer sechstägigen Kultur mit GM-CSF und IL-4 erzeugt. Am Tag 6 sind die DZ 24h lang nach Zugabe eines Standard-Reifungscocktail (IL-1β, IL-6, TNFα und PGE2) gereift (mDZ). Dann wurden die DZ ohne RNA, mit IKKα-EE-A16-RNA (aktiviert alternativen Signalweg), IKKβ-EEA10-RNA (aktiviert klassischen Signalweg) allein oder in Kombination (jeweils 15 µg RNA) elektroporiert. 24h nach der EP wurden die Überstände entnommen und durch einen "*Inflammation Cytometric Bead Array*" analysiert. Die Daten von 3 unabhängigen Spendern sind gezeigt.

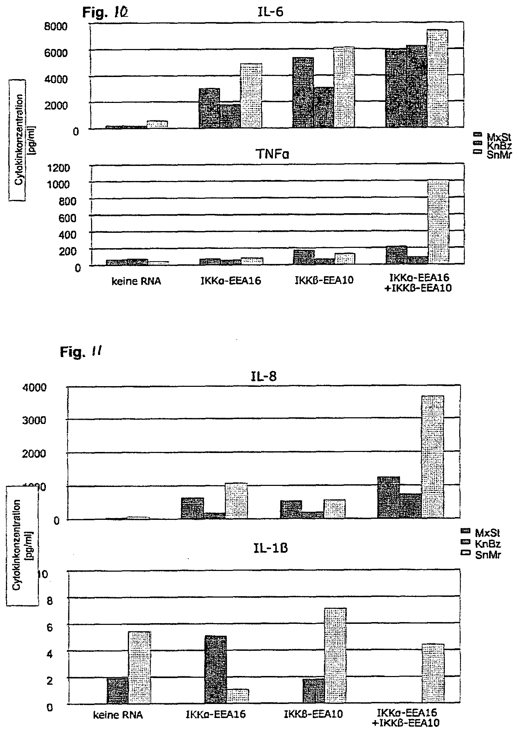

Fig. 10: Sekretion der Cytokine IL-6 und TNFα von DZ, die mit Komponenten des NFκB-Signalwegs elektroporiert wurden. DZ wurden aus Monocyten während einer sechstägigen Kultur mit GM-CSF und IL-4 erzeugt. Am Tag 6 sind die DZ 24h lang nach Zugabe eines Standard-Reifungscocktail (IL-1β, IL-6, TNFα und PGE2) gereift (mDZ). Dann wurden die DZ ohneRNA mit IKKα-EE-A16-RNA (aktiviert alternativen Signalweg), IKKβ-EEA10-RNA (aktiviert klassischen Signalweg) allein oder in Kombination (jeweils 15 µg RNA) elektroporiert. 24h nach der EP wurden die Überstände entnommen und durch einen "*Inflammation Cytometric Bead Array*" analysiert. Die Daten von 3 unabhängigen Spendern sind gezeigt.
Fig. 11: Sekretion der Cytokine IL-8 und IL-1β von DZ, die mit Komponenten des NFκB-Signalwegs elektroporiert wurden. DZ wurden aus Monocyten während einer sechstägigen Kultur mit GM-CSF und IL-4 erzeugt. Am Tag 6 sind die DZ 24h lang durch Zugabe eines Standard-Reifungscocktail (IL-1β, IL-6, TNFα und PGE2) gereift (mDZ). Dann wurden die DZ ohne RNA, mit IKKα-EE-A16-RNA (aktiviert alternativen Signalweg), IKKβ-EEA10-RNA (aktiviert klassischen Signalweg) allein oder in Kombination (jeweils 15 µg RNA) elektroporiert. 24h nach der EP wurden die Überstände entnommen und durch einen "*Inflammation Cytometric Bead Array*" analysiert. Die Daten von 3 unabhängigen Spendern sind gezeigt.

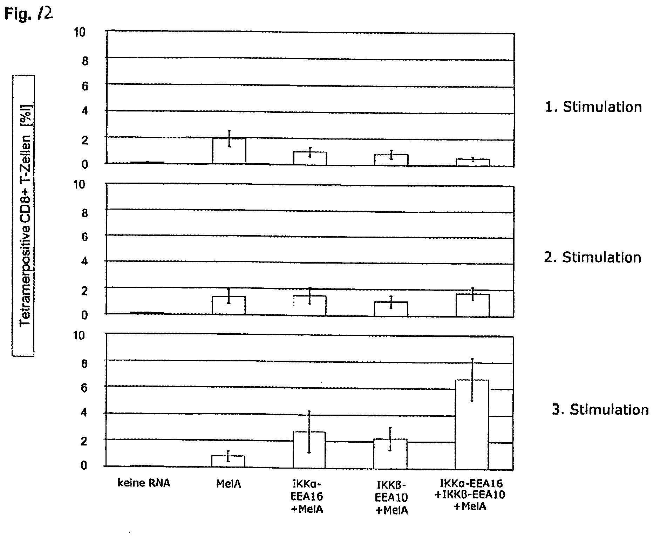

Fig. 12: Tetrameranfärbung der Stimulation von autologen T-Zellen mit DZ, die mit RNA von Komponenten des NFκB-Signalwegs elektroporiert wurden. Reife dendritische Zellen wurden ohne RNA, mit IKKα-EE-A16-RNA (aktiviert alternativen Signalweg), IKKβ-EEA10-RNA (aktiviert klassischen Signalweg) allein oder in Kombination (jeweils 15 µg RNA) elektroporiert. Ein Teil der DZ wurde mit RNA, die für MelanA (MelA) codierte, coelektroporiert. 4h nach der Elektroporation wurden autologe CD8+ T-Zellen mit diesen DZ im Verhältnis von 10:1 stimuliert. Eine Woche nach der Stimulation wurde die Zahl der antigenspezifischen T-Zellen durch Tetramer-Anfärbung analysiert, und der Phänotyp wurde durch CCR7- und CD45RA-Anfärbung identifiziert. T-Zellen wurden nach einer aktivierenden (1. Stimulation) und zwei erneuten Stimulationen (2. und 3. Stimulation) analysiert. Der Mittelwert von 5 unabhängigen Spendern ist mit dem Standardfehler des Mittelwerts angegeben

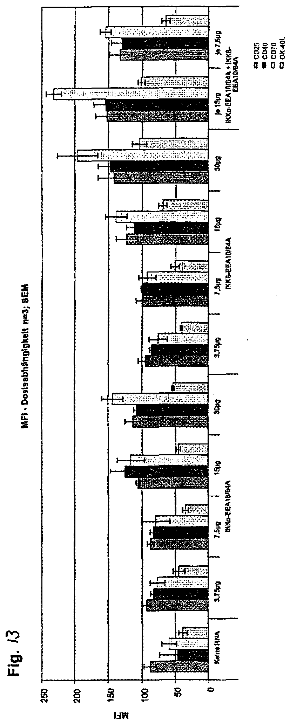

Fig. 13: Oberflächenmarker auf DZ, die mit Komponenten des NFκB-Signalwegs mit steigenden Konzentrationen der transfizierten RNA elektroporiert wurden. DZ wurden aus Monocyten während einer sechstägigen Kultur mit GM-CSF und IL-4 erzeugt. Am Tag 6 sind die DZ 24h lang nach Zugabe eines Standard-Reifungscocktail (IL-1β, IL-6, TNFα und PGE2) gereift (mDZ). Dann wurden die DZ ohneRNA mit IKKα-EE-A16-RNA (aktiviert alternativen Signalweg), IKKβ-EEA10-RNA (aktiviert klassischen Signalweg) allein oder in Kombination in steigenden Konzentrationen elektroporiert. Diese DZ wurden 24h nach der EP mit Antikörpern gegen CD25, CD40, CD70 und OX-40L angefärbt und durch FACS analysiert. Der Mittelwert von drei unabhängigen Spendern ist mit dem Standardfehler des Mittelwerts angegeben.

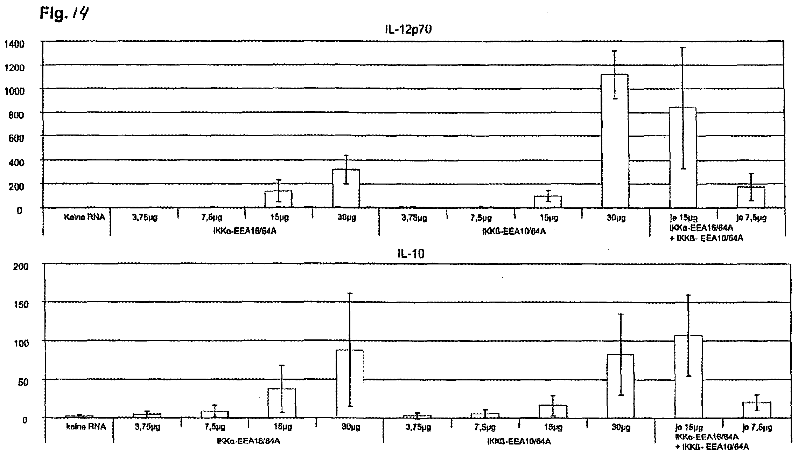

Fig. 14: Sekretion der Zytokine IL-12p70 und IL-10 durch DZ, die mit Komponenten des NFκB-Signalwegs mit steigenden Konzentrationen der transfizierten mRNA elektroporiert wurden. DZ wurden aus Monocyten während einer sechstägigen Kultur mit GM-CSF und IL-4 erzeugt. Am Tag 6 sind die DZ 24h lang nach Zugabe eines Standard-Reifungscocktail (IL-1β, IL-6, TNFα und PGE2) gereift (mDZ). Dann wurden die DZ ohne RNA, mit IKKα-EE-A16-RNA (aktiviert alternativen Signalweg), IKKβ-EEA10-RNA (aktiviert klassischen Signalweg) allein oder in Kombination (in steigenden Konzentrationen) elektroporiert. 24h nach der EP wurden die Überstände entnommen und durch einen "*Inflammation Cytometric Bead Array"* analysiert. Der Mittelwert von drei unabhängigen Spendern ist mit dem Standardfehler des Mittelwerts angegeben.

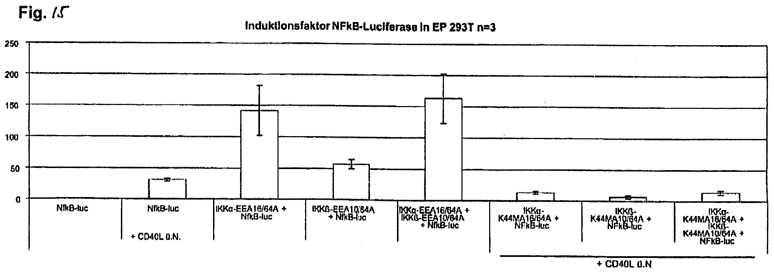

Fig. 15: Luciferase-Assay von 293T-Zellen, die mit Komponenten von NFκB-Signalwegen elektroporiert wurden. 293T-Zellen wurden mit Aktivatoren (IKKα-EEA16-RNA oder IKKβ-EEA10-RNA) oder Inhibitoren des NFκB-Signalwegs (IKKα-K44M-A16-RNA oder IKKβ-K44M-A10-RNA) allein oder in Kombination elektroporiert. Alle Zellen wurden mit Vektoren, die für Luciferase codierten einschließlich eines NFκB-Promotors coelektroporiert. Der NFκB-Signalweg eines Teils der Zellen wurde über Nacht mit löslichem CD40L aktiviert. Die Luciferase-Aktivität wurde 24h nach der Elektroporation gemessen.

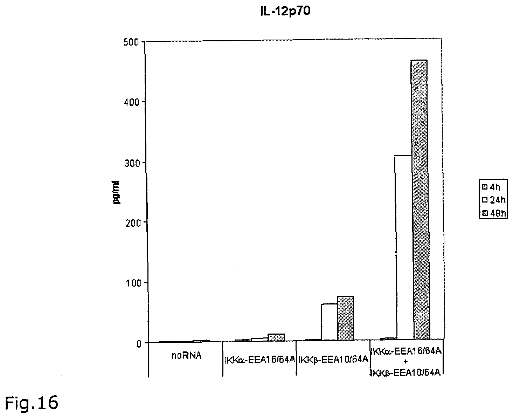

Fig. 16: Sekretion von IL-12p70 durch reife dendritischen Zellen, die mit RNA transfiziert wurden, die konstitutiv aktive IKK-Mutanten kodiert.

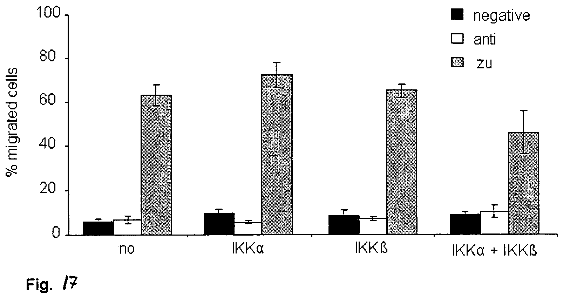

Fig. 17: Migration von reifen dendritischen Zellen, die mit RNA transfiziert wurden, die konstitutiv aktive IKK-Mutanten kodiert.

### Detaillierte Beschreibung der Erfindung

Die vorliegende Erfindung betrifft die in den Ansprüchen charakterisierten Ausführungsformen, nämlich dendritische Zellen (DZ), die durch RNA-Transfektion mit einer oder mehreren Nukleotidsequenzen, die mindestens ein mutiertes signalübertragendes Protein des NFκB-Signalweges kodieren, in ihrem NFκB-Signalweg manipuliert sind, deren Herstellung und Verwendung. Sie beruht auf dem Befund, dass DZ durch RNA-Transfektion und Expression von mutierten signalübertragenden Proteinen des NFκB-Signalweges in ihrem NFκB-Signalweg manipuliert werden können.

Der Begriff "dendritische Zellen" wird erfindungsgemäß wie im Stand der Technik verwendet. Sie sind in unreifem Zustand durch geringe Level an MHC-Proteinen und von kostimulatorischen B7-Molekülen gekennzeichnet, sowie der Fähigkeit zur Phagozytose und zur Pinozytose, sowie durch die Abwesenheit der Oberflächenmoleküle CD83 und CD25. In reifem Zustand sind sie unter anderem durch ein verändertes Muster an Oberflächenproteinen gekennzeichnet, wobei die Oberflächenexpression von einigen oder allen der folgenden Moleküle gesteigert wird: CD25, CD40, CD70, CD80, CD83, CD86, sowie MHC-Proteine. "Reife" unterscheiden sich von "unreifen" DZ unter anderem dadurch, dass erstere immunstimulatorisch aktiver sind, üblicherweise die Fähigkeit erhalten in vivo in den drainierenden Lymphknoten zu wandern, und verstärkt endogen exprimiertes und exogenes Antigen im MHC-Kontext zu präsentieren. Unter physiologischen Bedingungen sind nur "reife" DC in der Lage, naive T-Zellen zu aktivieren.

Der Begriff "RNA-Transfektion" wird erfindungsgemäß wie im Stand der Technik verwendet. Entsprechend bezeichnet die RNA-Transfektion das Einbringen von Fremd-RNA in eine eukaryotische Zelle, im Sinne der Erfindung eine DZ, vorzugsweise eine menschliche DZ. "Nukleotidsequenzen" umfassen erfindungsgemäß DNA und RNA. Die zu transfizierende RNA ist bevorzugt mRNA, die keine Introns enthält. Eine Definition von mRNA kann dem Stand der Technik entnommen werden (siehe "Molekulare Genetik", Knippers, 9. überarbeitete Auflage, Thieme Verlag, 2006). Die immunmodulatorische Effizienz der erfindungsgemäßen DZ kann weiter gesteigert werden, indem die mRNA stabilisiert wird. Dies kann beispielsweise durch Addition eines *cap*-Analogons während der *in vitro*-Transkription der mRNA erfolgen. Die Verwendung der sogenannten ARCA ("anti-reverses *cap*-Analogon")-Technologie führt zu einer zu 100% korrekten Orientierung des *cap* und damit zu einer weiteren Effizienzsteigerung (Stepinski et al., RNA 7(10), 2001, 1486-1495). Alternativ dazu kann die Stabilität der mRNA erhöht werden, wenn eine *cap*-Struktur enzymatisch auf die bereits *in vitro* synthetisierte mRNA aufgesetzt wird, wie beispielsweise in Tcherepanova et al., BMC Mol. Biol. (2008), 9:90 beschrieben. Die Stabilität der mRNA kann ferner durch Anfügen von nicht-translatierten Regionen (UTR), beispielsweise der ß-Globin-mRNA erhöht werden (vgl. z.B. Yu et al., Mol. Cell Biol. 21(17) (2001), 5879-5888).

Eine Verbesserung der Translationseffizienz mit einer erwarteten Verbesserung der immunmodulatorischen Eigenschaften der DZ kann ebenfalls durch die Verwendung der im vorherigen Absatz beschriebenen *capping-*Methoden erreicht werden, sowie auch durch bekannte Verfahren wie die Einfügung einer "Internal Ribosome Entry Site" (IRES) am 5'-Ende der *in vitro* translatierten RNA (Tan et al., Hum. Immunol. 69(1), 2008, 32-40. Die translatierte Ausbeute an Protein in Transfektionsexperimenten kann allgemein und so auch im Zusammenhang mit der vorliegenden Erfindung gesteigert werden, wenn die Länge des PolyA-Schwanzes verlängert wird. Diese Technologie führt zu noch besseren Ergebnissen, wenn sie zusammen mit der ARCA-Technik angewandt wird (Mockey et al., Biochem. Biophys, Res. Commun. 340(4) (2006), 1062-1068).

In Bezug auf die vorliegende Erfindung ist ein "mutiertes signalübertragendes Protein des NFκB-Signalweges" als Protein definiert, das eine Komponente der bekannten Signalkaskade ist, die zur Aktivierung von NFκB und der nachfolgenden Translokation dieses Proteins in den Zellkern führt. Ferner umfasst sind von diesem Begriff erfindungsgemäß Proteine, die in modulatorischer Weise mit Komponenten der Signalkaskade interagieren und diese in ihrer Aktivität beeinflussen. All diese Proteine weisen gegenüber entsprechenden Wildtyp-Proteinen Veränderungen (Mutationen) auf. Die entsprechenden Mutationen sind unter anderem durch Deletionen, Extensionen oder bevorzugt der Austausch einer oder mehrerer Aminosäuren definiert.

Die erfindungsgemäße Definition des NFκB-Signalwegs umfasst den klassischen und den alternativen Signalweg. Der klassische Signalweg wird durch mikrobiologische und virale Infektionen oder durch Zytokine aktiviert. In diesem Zusammenhang induziert der IKK-Komplex, bestehend aus IKKα, IKKβ und NEMO, durch Phosphorilierung den Abbau von IκB, worauf der Transkriptionsfaktor NFκB in den Zellkern transloziert und diverse Zielgene aktiviert. Der alternative NFκB-Signalweg ist unabhängig von IKKβ und NEMO. Hier interagiert IKKα mit p100 (NFκB2), das zu seiner p52 Form prozessiert wird und zusammen mit RelB in den Zellkern transloziert und Zielgene aktiviert.

"Manipulationen" des NFκB-Signalwegs in Bezug auf die vorliegende Erfindung können durch eine veränderte Aktivität von dendritischen Zellen gemessen werden. Diese umfasst Veränderungen der Sekretion von IL12p70, der Sekretion von IL-10, der Migration oder der Expression verschiedener Induktionsfaktoren, wie z.B. OX-40L oder CD25. Bevorzugt umfasst ist die (vermehrte) Sekretion von IL12p70 mit vorzugsweise mindestens 5-fach, stärker bevorzugt mindestens 10-fach, noch stärker bevorzugt mindestens 30-fach und am meisten bevorzugt mindestens 50-fach erhöhten Werten aus dendritischen Zellen, die mit konstitutiv-aktiven Mutanten von IKKα und/oder IKKβ transfiziert wurden, gegenüber mit Kontrol-RNA transfizierten dendritischen Zellen oder nicht transfizierten dendritischen Zellen, die bevorzugt reife dendritische Zellen sind. Weiter bevorzugt ist die Sekretion von IL-10 mit mindestens 5-fach, weiter bevorzugt mindestens 10-fach und am meisten bevorzugt mindestens 30-fach erhöhten Werten aus dendritischen Zellen, vorzugsweise unreifen dendritischen Zellen, die vorzugsweise mit konstitutiv-aktiven Mutanten von IKKα und/oder IKKβ transfiziert wurden und gegenüber nicht transfizierten oder mit Kontroll-RNA transfizierten dendritischen Zellen verglichen werden. Bevorzugt sein kann auch eine hohe IL-12p70-Sekretion bei gleichzeitig niedriger IL-10-Sekretion mit einem Mengenverhältnis von IL-12p70 zu IL-10 von vorzugsweise mindestens 3, stärker bevorzugt mindestens 5, noch stärker bevorzugt mindestens 10 und am meisten bevorzugt mindestens 20 durch bevorzugt reife dendritische Zellen, die mit konstitutiv aktiven Mutanten von IKKα und/oder IKKβ transfiziert wurden.

Über die RNA-Transfektion von dendritischen Zellen konnten die Erfinder in der vorliegenden Erfindung überraschenderweise zeigen, dass durch RNA-Transfektion veränderte dendritische Zellen über den NFκB-Signalweg in ihrer Funktionsweise manipuliert werden können. Die RNA-Transfektion verschiedener RNAs in dendritische Zellen kann entweder zu einer Immunstimulation oder einer Toleranzinduktion bzw. einer Supprimierung immunologischer Antworten führen. Die transfizierten RNAs können die Sekretion von IL-12p70 verstärken und folglich zu einer Immunstimulation führen. Ein weiterer überraschender Effekt der vorliegenden Erfindung beruht darauf, dass identische RNAs, z.B. RNAs, die konstitutiv aktivierende Mutanten von IKKα oder IKKβ kodieren, verschiedene Effekte auf die Immunstimulation von dendritischen Zellen haben, abhängig vom Zeitpunkt ihrer RNA-Transfektion (siehe Fig 2a und 2b). Erfindungsgemäß wird bei Transfektion von reifen DZ mit RNAs, deren Transkription zu Proteinen führt, bei denen die Serinreste, die bei physiologischer Aktivierung von IKK phosphoryliert werden und dadurch die Kinaseaktivität von IKK vermitteln, durch Glutaminreste ausgetauscht sind, eine Immunstimulation beobachtet. Dadurch kann, beispielsweise bei gleichzeitiger Präsentation von Tumorantigenen durch die DZ, eine effektive Killer-T-Zell-Aktivierung erreicht werden, was man sich nach Gabe der transfizierten (autologen) DZ in den Patienten bei der Bekämpfung von Tumoren zunutze machen kann. Dieses Prinzip kann entsprechend bei anderen Krankheiten angewendet werden. Wird die RNA-Transfektion dagegen mit unreifen DZ durchgeführt, erwartet man erfindungsgemäß eine immunsupprimierende Wirkung, da derartige DZ große Mengen des immunsuppressiven IL-10 sekretieren. Beide Alternativen stellen bevorzugte Ausführungsformen der Erfindung dar. Es konnte überraschenderweise auch gezeigt werden, dass die erfindungsgemäßen reifen DZ zur Migration befähigt sind (siehe Fig. 5). Eine derartige Kontrolle über die immunologische Aktivität von dendritischen Zellen stellt für die medizinische Anwendung, z.B. in Form von Vakzination mit dendritischen Zellen zur Behandlung von Krebspatienten, einen enormen Fortschritt dar.

Erfindungsgemäß können die genannten immunmodulatorischen, insbesondere Antigen-präsentierenden Eigenschaften der DZ noch weiter verbessert werden, wenn in die DZ neben der das/die genannten mutierte(n) signalübertragenden Protein kodierenden RNA inhibitorische RNAs wie siRNAs eingeschleust oder dort exprimiert werden, um mRNAs zu inaktivieren, die immunsuppressive Proteine wie unter anderem A20, IL-10, TGF kodieren. Ein derartiges Verfahren ist in Breckpot et al., J. Immunol. 182(2) (2009), 860-870 beschrieben. Eine höhere effizientere Induktion von antigenspezifischer cytolytische Aktivität durch die DZ als Antigenpräsentierende Zelle wird auch erwartet, wenn in dieser Immun-Proteasom-mRNA inaktiviert wird, beispielsweise durch siRNAs (Dannull et al., Blood 110(13) (2007), 4341-4350). Eine verbesserte Wirkung der DZ-mRNA-Vakzine, beispielsweise in der Krebsbekämpfung, wird auch nach Stimulation der DZ durch einzel- oder doppelsträngige RNA-Sequenzen erwartet (vgl. z.B. Diebold et al., Science 303 (2004), 1529-1531).

Geeignete bevorzugte mutierte signalübertragende Proteine des NFκB-Signalweges sind dabei Mutanten des Inhibitors-von-kappa-Kinasen IKK und anspruchsgemäß konstitutiv-aktive IKKα oder IKKβ Mutanten. Für die Herstellung der DZ gemäß Aspekt (1) der Erfindung wurden experimentell mehrere konstitutiv aktive oder dominant negative Mutanten verschiedener IKKs hergestellt. Konstitutiv-aktive IKKα und IKKβ Mutanten sind vorzugsweise dabei solche, die, ausgehend von den entsprechenden Wild-Typ Sequenzen vorzugsweise von SEQ ID NO:1 bzw. 4, eine oder mehrere Substitutionen von Ser durch Glu im aktiven Zentrum aufweisen.

Dabei sind solche IKKα Mutanten bevorzugt, in denen eine oder mehrere der Aminosäurereste Ser176 und Ser180 des IKKα Wild-Typs von SEQ ID NO:1 durch Glu ersetzt ist, besonders bevorzugt die Aminosäurereste Ser176 und Ser180 des IKKα Wild-Typs von SEQ ID NO:1 durch Glu ersetzt sind, und optional einer oder mehrere der destabilisierenden C-terminalen Serin- und Threoninreste, vorzugsweise destabilisierende Serin- und Threoninreste in den Positionen 661, 662, 665, 669, 670, 676, 679, 680, 686, 687, 693, 695, 699, 705, 706, 721 und 722 und in einer weniger bevorzugten Form 661, 662, 665, 669, 670, 676, 679, 680, 686, 687, 692, 694, 698, 704 und 705 des Wild-Typs von SEQ ID NO:1 durch Alaninreste ersetzt sind. Alle genannten IKK Mutanten, welche durch die Einfügung von Alaninresten gekennzeichnet sind führen zu einer Stabilisation des Proteins und sind ferner dadurch gekennzeichnet, dass sie die Wirkung der gesteigerten oder hemmenden Aktivität jeweils verstärken. Die Einführung der stabilisierenden Alaninreste in die Proteine stellt bei allen erfindungsgemäßen Ausführungsformen darüber hinaus bevorzugte Ausführungsformen dar. Bevorzugt sind mindestens zwei, vorzugsweise mindestens drei, stärker bevorzugt mindestens vier, stärker bevorzugt mindestens acht und, anspruchsgemäß, sämtliche der genannte Reste durch Alaninreste ausgetauscht. Alle möglichen Permutationen, auch wenn sie hier nicht genannt sind, sind explizit vom Offenbarungsgehalt dieser Spezifikation so umfasst, als wären sie einzeln genannt. Weiterhin sind solche IKKβ Mutanten bevorzugt, in denen eine oder mehrere der Aminosäurereste Ser177 und Ser181 des IKKβ Wild-Typs vorzugsweise von SEQ ID NO:4 durch Glu ersetzt ist, besonders bevorzugt die Aminosäurereste Ser177 und Ser181 des IKKβ Wild-Typs von SEQ ID NO:4 durch Glu ersetzt sind, und optional eines oder mehrere der destabilisierenden C-terminalen Serin- und Threoninreste, vorzugsweise solche destabilisierende Serin- und Threoninreste in den Positionen 670, 672, 675, 679, 682, 689, 692, 695, 697 und 705 des Wild-Typs von SEQ ID NO:4 durch Alaninreste ersetzt sind.

Die Erfindung betrifft solche konstitutiv-aktiven IKKα und IKKβ Mutanten, die Aminosäurereste 25 bis 769 von SEQ ID NO:2 bzw. die Aminosäurereste 18 bis 773 von SEQ ID NO:5 umfassen, vorzugsweise die Sequenz von SEQ ID NO:2 bzw. SEQ ID NO:5 aufweisen, oder wobei die kodierende RNA Sequenz die SEQ ID NO:3 oder 6 enthält. Außerdem ist jede mRNA, die eine Sequenz enthält, die durch stumme Mutationen aus der Sequenz SEQ ID NO:3 oder 6 hervorgehen kann, wie unter anderem durch Codon-Optimierung, vom Offenbarungsgehalt dieser Spezifikation umfasst.
Die ebenfalls hierin beschriebenen hemmenden IKKα und IKKβ Mutanten sind dabei solche, die, ausgehend von den entsprechenden Wild-Typ Sequenzen von SEQ ID NO:1 bzw. 4, eine Substitutionen von Lys durch Met aufweisen. Dabei sind solche IKKα Mutanten bevorzugt, in denen der Aminosäurerest Lys44 des IKKα Wild-Typs von SEQ ID NO:1 durch Met ersetzt ist, und optional einer oder mehrere der destabilisierenden C-terminalen Serin- und Threoninreste, vorzugsweise destabilisierende Serin- und Threoninreste in den Positionen 661, 662, 665, 669, 670, 676, 679, 680, 686, 687, 693, 695, 699, 705, 706, 721 und 722 des Wild-Typs von SEQ ID NO:1 durch Alaninreste ersetzt sind. Weiterhin sind solche IKKβ Mutanten bevorzugt, in denen der Aminosäurerest Lys44 des IKKβ Wild-Typs von SEQ ID NO:4 durch Met ersetzt ist, und optional einer oder mehrere der destabilisierenden C-terminalen Serin- und Threoninreste, vorzugsweise solche destabilisierende Serin- und Threoninreste in den Positionen 670, 672, 675, 679, 682, 689, 692, 695, 697 und 705 des Wild-Typs von SEQ ID NO:4 durch Alaninreste ersetzt sind.
Besonders bevorzugt sind solche hemmenden IKKα und IKKβ Mutanten, die Aminosäurereste 24 bis 768 von SEQ ID NO:7 bzw. die Aminosäurereste 24 bis 779 von SEQ ID NO:9 umfassen, vorzugsweise die Sequenz von SEQ ID NO:7 bzw. SEQ ID NO:9 aufweisen, oder die RNA Sequenz von SEQ ID NO:8 oder 10 aufweisen, oder deren RNA-Sequenz sich durch stumme Mutationen in die RNA-Sequenz von SEQ ID NO:8 oder 10 umwandeln lässt.
Eine weitere besonders bevorzugte Ausführungsform, wie oben erwähnt, betrifft erfindungsgemäße dendritische Zellen, wobei die DZ (i) reife DZ sind; und/oder; (ii) NFκB-aktivierte DZ sind, die IL-12p70 produzieren; und/oder (iii) NFκB-aktivierte DZ sind, die IL-10 produzieren; und/oder (iv) weiterhin mit einem oder mehreren Targetantigen beladen sind.
Die Definition von "Targetantigen" umfasst in der vorliegenden Erfindung Peptidketten, die z.B. an den major histocompatibility complex (MHC) gebunden sind, und an der Zelloberfläche von dendritischen Zellen, T-Zellen präsentiert werden. Diese können sich unter anderem von Tumorantigen ableiten wie z.B. MelanA, GP100, Mitgliedern der MAGE-Familie, aber auch mutierte Tumorantigen wie BRAF-V600E und GNAQ-Q209L. Es können aber auch Quellen für nicht definierte Antigen verwendet werden wie Tumorlysat oder aus dem Tumor isolierte mRNA. Jedes virale Protein kann ebenfalls Antigenquelle sein, wie zum Beispiel HIV-1-NEF oder Influenza Matrix Protein.
Die oben genannten Mutanten können durch RNA-Transfektion von entsprechenden mRNA-Molekülen in DZ exprimiert werden. Die RNA-Transfektion stellt keine genetische Veränderung der DZ dar, und ist somit vom klinischen Standpunkt aus unbedenklich. Wurden DZ mit einer konstitutiv aktiven IKK-Mutante transfiziert, nachdem sie mit den Zytokinen IL-1β, IL-6, TNFα und PGE₂ inkubiert ("gereift") wurden, so begannen diese, das proinflammatorische Zytokin IL-12p70 zu sezernieren (Fig. 2a), dem in der Induktion von robusten, lang anhaltenden Immunantworten eine entscheidende Rolle zugeschrieben wird. Bezüglich der Reifung der DZ können neben IL-1beta, IL-6, TNF und PGE2 alternativ oder ergänzend weitere Substanzen zur Reifung der DZ verwendet werden, einschließlich aber nicht beschränkt auf: IFN-alpha, -beta, -gamma, künstliche und natürliche TLR-Agonisten, wie unter anderem polyI:C, CpG, LPS, Flagellin, oder lösliche und oberflächengebundene Substanzen die spezifisch Oberflächenrezeptoren der DZ binden.
Erfolgte die RNA-Transfektion mit den aktivierenden Mutanten jedoch zu Beginn der Reifung (also bei unreifen DZ), entstanden DZ, die große Mengen des unter bestimmten Bedingungen immunsuppressiven Zytokins IL-10 abgaben (Fig. 2b). Des Weiteren zeigten verschiedene Reifungsmarker auf den DZ eine erhöhte Expression nach RNA-Transfektion der beschriebenen RNAs, auch solche, denen eine Rolle in der Kommunikation der DZ mit anderen Zellen des Immunsystems zugesprochen wird (Fig. 3). Besonders das Oberflächenmolekül CD70 ist hier von Interesse, da ihm eine Rolle in der Induktion langlebiger Gedächtnis-T-Zellen zugesprochen wird (Fig. 3, unten). Der Phänotyp langlebiger Gedächtnis-T-Zellen wurde eingangs beschrieben. Wurden die so behandelten DZ verwendet, um autologe CD8⁺ T-Zellen wiederholt zu stimulieren, so wurde beobachtet, dass die NFκB-Aktivierung die DZ befähigte, die T-Zellen bei Restimulation weiter zu expandieren, wobei der T-Zell-Phänotyp der Effektor-Gedächtnis-Zellen verstärkt repräsentiert war (Fig. 4). Ein weiterer kritischer Faktor bei der Herstellung immunogener DZ ist deren Migrationsfähigkeit, die meistens bei IL-12-sekretierenden DZ verloren geht. Die mit den konstitutiv aktiven NFκB-Mutanten transfizierten DZ waren überraschenderweise in der Lage, ebenso effizient gegen das Chemokin MIP-3ß zu migrieren, wie DZ, die mit einer Kontroll-RNA elektroporiert wurden (Fig. 5). Somit stellt die RNA-Transfektion von DZ mit mRNA, die für funktionelle Mutanten des NFκB-Signalweges kodiert, eine neue, innovative Methode zur Erzeugung immunogener oder tolerogener DZ dar, deren konkrete klinische Anwendung sich abzeichnet.

In weiteren bevorzugten Ausführungsformen der Erfindung ist die DZ mit mRNAs, die CD70, gegebenenfalls in Kombination mit solchen, die caTLR4 und CD40-Ligand oder/ OX40L kodieren, co-transfiziert. Alle in dieser Anmeldung genannten Moleküle, die in die vorzugsweise menschlichen DZ eingeführt werden, kodieren vorzugsweise Moleküle, die den im Menschen vorkommenden in ihrer Aminosäuresequenz entsprechen, oder sich von diesen ableiten.

Das Verfahren zur *ex-vivo* Herstellung von NFκB-Signalweg manipulierten DZ gemäß Aspekt (2) der Erfindung umfasst die RNA-Transfektion von unreifen oder reifen DZ mit einer oder mehreren Nukleotidsequenzen, die ein wie vorstehend beschriebenes mutiertes signalübertragendes Protein des NFκB-Signalweges kodieren. Es ist dabei bevorzugt, dass die RNA-Transfektion durch Elektroporation erfolgt (andere dem Fachmann bekannte Verfahren wie Lipofektion usw. sind ebenfalls anwendbar). Eine bevorzugte Ausführungsform des Elektroporationsverfahrens ist die von Tuyaerts *et al.* beschriebene Methode, die sich besonders gut für klinische Anwendungen eignet (Cancer Gene Ther. 10(9) (2003), 696-706). In einer weiteren bevorzugten Ausführungsform wird als RNA-Transfektionstechnologie die Nukleofection (proprietäre Technologie der Fa. Amaxa) eingesetzt (vgl. z.B. Melhem et al., Clin. Vaccine Immunol. 15(9) (2008), 1337-1344). Bevorzugte Konzentrationen zur Transfektion von RNA durch Elektroporation umfassen insbesondere etwa 1 µg/100 µl bis etwa 100 µg/100 µl, weiter bevorzugt 2 µg/100 µl bis 50 µg/100 µl, und am meisten bevorzugt etwa 20 µg bis etwa 40 µg /100 µl. mRNA-Transfektion kann neben der erwähnten Elektroporation, die sowohl durch einen Rechteckwellenimpuls, als auch durch einen exponentiell abfallenden Puls erfolgen kann, durch verschiedene Reagenzien zur mRNA-Transfektion erreicht werden. Exemplarisch seien hier geladene und ungeladene Lipide genannt, mit deren Hilfe DZ mit mRNA transfiziert werden können.
Das erfindungsgemäße Verfahren kann, im Falle der RNA-Transfektion von unreifen DZ, weiterhin das Behandeln mit einem Reifungsstimulus umfassen. Weiterhin bevorzugte Ausführungsformen umfassen das Beladen der DZ mit einen Targetantigen und/oder (iii) die Kryokonservierung von reifen DZ.
Als "Reifungsstimulus" werden hier Moleküle und Molekülkombinationen definiert, unter deren Mithilfe unreife dendritische Zellen zu reifen dendritischen Zellen werden. Eine hier bevorzugte Molekülkombination besteht aus IL-1β, IL-6, TNFα und PGE2.
In Bezug auf die vorliegende Erfindung ist "Kryokonservierung" als das Aufbewahren von Zellen durch Einfrieren bei Temperaturen unter - 75°C verstehen.

Die Zusammensetzung, pharmazeutische Zusammensetzung oder Medikament gemäß Aspekt (3) der Erfindung kann optional pharmazeutisch geeignete Hilfs- und Trägerverbindungen aufweisen. Für die pharmazeutische Verwendung ist dabei bevorzugt, dass die DZ autologe DZ sind.
Eine "pharmazeutische Zusammensetzung" oder ein "Medikament" beinhaltet die erfindungsgemäßen dendritischen Zellen und eine oder mehrere Komponenten, die Patienten, z.B. in Form einer Vakzination zur Behandlung von Krebs oder HIV, verabreicht werden. Verfahren und Mittel zur Formulierung einer pharmazeutischen Zusammensetzung sind dem Fachmann bekannt und beispielsweise Ansel et al., "Pharmaceutical Dosage Forms and Drug Delivery Systems", 7te Auflage, Lippincott Williams & Wilkins Publishers, 1999 zu entnehmen. Die pharmazeutische Zusammensetzung oder das Medikament kann einem Individuum in einer geeigneten Dosis verabreicht werden. Die Verabreichung kann insbesondere parenteral erfolgen, z.B. intravenös, intraperitoneal, subcutan oder intramuskulär oder über einen Katheter an einer Stelle in einer Arterie. Präparate für eine parenterale Verabreichung umfassen sterile wässrige oder nicht-wässrige Lösungen, Suspensionen und Emulsionen. Beispiele für nicht-wässrige Lösungsmittel sind Propylenglykol, Polyethylenglykol, pflanzliche Öle wie z.B. Olivenöl, und organische Esterverbindungen wie z.B. Ethyloleat, die für Injektionen geeignet sind. Wässrige Träger umfassen Wasser, alkoholisch-wässrige Lösungen, Emulsionen, Suspensionen, Salzlösungen und gepufferte Medien. Parenterale Träger umfassen Natriumchlorid-Lösungen, Ringer-Dextrose, Dextrose und Natriumchlorid, Ringer-Laktat und gebundene Öle. Intravenöse Träger umfassen z.B. Flüssigkeits-, Nährstoff- und Elektrolyt-Ergänzungsmittel (wie z.B. solche, die auf Ringer-Dextrose basieren). Die pharmazeutische Zusammensetzung oder das Medikament kann außerdem Konservierungsmittel und andere Zusätze umfassen, wie z.B. antimikrobielle Verbindungen, Antioxidantien oder Komplexbildner. Des weiteren können, abhängig von der beabsichtigten spezifischen Verwendung, andere Wirkstoffe wie z.B. Interleukine, Wachstumsfaktoren, Differenzierungsfaktoren, Interferone, chemotaktische Proteine oder ein unspezifisches immunmodulatorisches Agens enthalten sein.

Die Art der Dosierung wird vom behandelnden Arzt entsprechend den klinischen Faktoren bestimmt. Es ist dem Fachmann bekannt, dass die Art der Dosierung von verschiedenen Faktoren abhängig ist, wie z.B. der Körpergröße bzw. dem Gewicht, der Körperoberfläche, dem Alter, dem Geschlecht oder der allgemeinen Gesundheit des Patienten, aber auch von dem speziell zu verabreichenden Mittel, der Dauer und Art der Verabreichung, und von anderen Medikamenten, die möglicherweise parallel verabreicht werden. Eine typische Dosis kann z.B. in einem Bereich zwischen 5 Millionen und 50 Millionen DZ pro Verabreichung liegen. Die zeitliche Staffelung wiederholter Verabreichungen erfolgt üblicherweise zuerst mit geringeren Abständen im Bereich von ein bis 2 Wochen und später können die Intervalle bis auf 6 Monate ausgedehnt werden. In den bisherigen Studien wurden DZ gewöhnlich intradermal, subkutan und intravenös injiziert.

"Geeignete Hilfs- und Trägerverbindungen" umfassen Komponenten, auf die die erfindungsgemäßen Zellen aufgebracht oder eingebracht werden können und beispielsweise die Zellen schützen. Beispiele für geeignete pharmazeutisch verträgliche Hilfs- und Trägerverbindungen sind dem Fachmann bekannt und umfassen z.B. Phosphat-gepufferte Kochsalzlösungen, Wasser, Emulsionen, wie z.B. Öl/Wasser-Emulsionen, verschiedene Arten von Netzmittel oder Detergenzien, sterile Lösungen, etc. Pharmazeutische Zusammensetzung oder Medikamente, die solche Träger umfassen, können mittels bekannter konventioneller Methoden formuliert werden.

"Autologe" dendritische Zellen sind als körpereigene Patientenzellen oder aus körpereigenen Patientenzellen entstandene Zellen zu verstehen.

Bei der Verwendung der erfindungsgemäßen DZ zur Stimulation autologer CD8⁺ T-Zellen *ex vivo* gemäß Aspekt (4) der Erfindung, werden vorzugsweise (i) NFκB-aktivierte DZ für passive T-Zell-Übertragung und Erzeugung eines T-Zell-Klons (einschließlich der anschließenden TCR-Isolierung), und (ii) NFκB-reprimierte DZ zur Expansion von Treg für die Behandlung von Allergie, chronischer Entzündung Autoimmunität und Transplantatsabstoßung eingesetzt.
"CD8⁺ T-Zellen" sind durch die Anwesenheit des Oberflächenmarkers CD8 gekennzeichnet und gehören einer Subgruppe von T-Lymphozyten an, die in der Lage sind, infizierte somatische Zellen oder Tumorzellen abzutöten.

Unter passiver Immunisierung oder "passiver T-Zell-Übertragung" ist der Transfer von außerhalb des Empfängers generierten immunologischen Effektoren bzw. T-Zellen zu verstehen. Der Empfänger muss die Immunantwort also nicht selbst aktiv ausbilden, sondern erhält diese von außen, ist also selbst passiv. Die passive Immunisierung mit T-Zellen, also die passive T-Zell-Übertragung, wird auch als adaptiver T-Zell-Transfer bezeichnet.
In Bezug auf die vorliegende Erfindung ist ein "T-Zell-Klon" eine von einer T-Zelle eines Patienten abgeleitete und in Kultur haltbare Population von Zellen die auf eine individuelle T-Zelle zurück geht.
"Expansion von Treg" bezeichnet die Kultivierung und Vermehrung von regulatorischen T-Zellen, die die Funktionen anderer T-Zellen unterdrücken kann. Als "Allergie" wird eine überschießende Abwehrreaktion des Immunsystems auf bestimmte und normalerweise harmlose Umweltstoffe (Allergene) bezeichnet. "Autoimmunität" kann als überschießende Abwehrreaktion des Immunsystems auf körpereigenes Gewebe definiert werden.
"TCR-Isolierung" bezeichnet eine Methode zur Gewinnung der Nukleotidsequenzen aus einem T-Zell-Klon die den T-Zell-Rezeptor (TCR) kodieren. Methoden dazu können dem Stand der Technik entnommen werden.

Bei der Verwendung der erfindungsgemäßen DZ zur Herstellung eines Medikaments zur Behandlung von Erkrankungen in einem Patienten gemäß Aspekt (5) der Erfindung und dem Verfahren zur Behandlung von Krebs, Infektions- oder Autoimmunerkrankungen in einem Patienten, umfassend das Verabreichen der erfindungsgemäßen DZ an den Patienten gemäß Aspekt (7) der Erfindung werden vorzugsweise NFκB-aktivierte DZ zur DZ-basierten Impfung (insbesondere in Abwesenheit von Helferepitopen oder funktionellen Helferzellen, und wenn die Anwendung starker Adjuvantien nicht möglich ist), zur therapeutischen Impfung gegen Krebs oder Infektionskrankheiten (einschließlich HIV) und als präventiver Impfstoff, und NFκB-reprimierte DZ zur Induktion von Toleranz in vivo, und zur Behandlung von Allergie, chronischer Entzündung, Autoimmunität und Transplantatsabstoßung eingesetzt.
Die "DZ-basierte Impfung" beschreibt eine Methode zur Verabreichung von dendritischen Zellen an Patienten, vorzugsweise durch eine Injektion. "Helferepitope" werden im MHC/HLA-Klasse II-Kontext präsentiert und können die T-Zell Proliferation und die Synthese von Cytokinen induzieren.

"Funktionelle Helferzellen" sind eine Gruppe der T-Lymphozyten im Blut, die eine Helferfunktion haben. Sie werden anhand der von ihnen ausgeschütteten Zytokine in zwei wichtige Untergruppen eingeteilt. Eine Untergruppe ist an der zellulären Immunantwort beteiligt, während die andere Untergruppe an der humoralen Immunantwort beteiligt ist.
"Adjuvantien" sind Hilfsstoffe, die die Wirkung eines Reagenz oder eines Arzneimittels, insbesondere die Immunantwort verstärken.
"Krebs" bezeichnet einen malignen Tumor oder eine maligne Leukämie. "Infektionskrankheiten" sind durch einen Erreger, z.B. Viren, Bakterien, Pilze oder andere Mikroorganismen hervorgerufene Erkrankungen.
Die "Induktion von Toleranz in vivo" bezeichnet in der vorliegenden Anmeldung die Repression einer immunologischen Abwehrreaktion in Patienten, vorzugsweise einem menschlichen Patienten.

Bei dem Verfahren zur Expansion von T Zellen einschließlich der Stimulation autologer CD8⁺ T-Zellen *ex vivo*, umfassend Stimulieren der T Zellen mit erfindungsgemäßen DZ gemäß Aspekt (6) der Erfindung, werden vorzugsweise NFκB-aktivierte DZ zur T-Zell-Expansion für T Zellen zur passiven T-Zell-Übertragung und Erzeugung eines T-Zell-Klons (z.B. für anschließende TCR-Isolierung), und NFκB-reprimierte DZ zur Expansion von Treg für die Behandlung von Allergie, chronischer Entzündung, Autoimmunität und Transplantatsabstoßung eingesetzt.
Eine gewünschte verbesserte T-Zell-Proliferation und damit eine verbesserte medizinische Wirkung kann auch durch gleichzeitige Verabreichung, oder gleichzeitige Expression in der DZ via transfizierte mRNA(s), von Antikörpern, vorzugsweise gegen CTLA-4, PD-L1, PD-L2, PD1, oder von einem agonistischen anti-GITR-Antikörper erzielt werden ((vgl. z.B. Leach et al., Science 271 (1996), 1734-1736; Quezada et al., J. Clin. Invest, 116(7) (2006), 1935-1945).

Eine weitere Ausführungsform betrifft ein Verfahren zur Behandlung von Erkrankungen in einem Patienten, umfassend das Verabreichen der erfindungsgemäßen DZ an den Patienten, wobei vorzugsweise (i) NFκB-aktivierte DZ zur DZ-basierten Impfung (insbesondere in Abwesenheit von Helferepitopen oder funktionellen Helferzellen, und wenn die Anwendung starker Adjuvantien nicht möglich ist), zur therapeutischen Impfung gegen Krebs oder Infektionskrankheiten (einschließlich HIV) und/oder als präventiver Impfstoff, und (ii) NFκB-reprimierte DZ zur Induktion von Toleranz in vivo und zur Behandlung von Allergie, chronischer Entzündung, Autoimmunität und Transplantatsabstoßung eingesetzt werden.

Im Folgenden werden mögliche therapeutische Anwendungen von dendritischen Zellen diskutiert. Eine mögliche Strategie zur Behandlung eines Krebspatienten beinhaltet die Gewinnung von Monozyten aus dem Blut des Patienten, die Differenzierung dieser Monozyten zu dendritischen Zellen (DZ) mittels GM-CSF und IL-4, oder gleichwirkenden Zytokinen; die Reifung der DZ mittels IL-1beta, IL-6, TNF und PGE2, oder gleichwirkenden Reifungsstimulatoren; die Elektroporation der DZ mit mRNA, deren Sequenz für eine, oder beide der NFkappaB-aktivierenden Mutanten von IKK-alpha und IKK-beta kodiert; die Beladung der DZ mit einem oder mehreren Tumor-assoziierten Antigen(en) durch entweder die Ko-Elektroporation einer mRNA, deren Sequenz für diese(s) kodiert oder durch exogene Beladung der DZ mit einem oder mehreren synthetischen Peptid(en), das an HLA-Moleküle der DZ binden kann; die Kryokonservierung der DZ in geeigneten Portionen; Kontrolle der Qualität der DZ durch Bestimmung der IL-12p70-Sekretion; die intravenöse oder intra- oder subdermale Injektion der DZ in den Patienten in mehreren gestaffelten Gaben.
Bezüglich der Herstellung der DZ wird erfindungsgemäß die direkte Gewinnung von DZ aus frischem oder kryokonserviertem Patentenmaterial ins Auge gefasst, einschließlich aber nicht beschränkt auf Blut oder Blutzellen oder anderem Gewebe des Patienten durch magnetische oder fluoreszenzaktivierte Zellsortierung oder die Differenzierung der DZ aus Knochenmarksstammzellen die, zum Beispiel, über den Stammzellmarker CD34 aufgereinigt wurden. Zur Differenzierung der Monozyten und der Stammzellen in die DZ können neben GM-CSF und IL-4 weitere Substanzen verwendet werden einschließlich aber nicht beschränkt auf: Flt3-Ligand, IL-15, IFN-alpha, TNF.

Als Antigenquellen zur Antigenbeladung kann unter anderem autologes und allogenes Tumormaterial verwendet werden, sowie daraus gewonnene und amplifizierte mRNA, außerdem enzymatisch hergestellte mRNA, die für Tumorantigene oder Teile davon kodiert. HLA-bindende Peptide, die sich von Tumorantigenen ableiten, können direkt auf die HLA-Moleküle der DZ geladen werden. Gentechnisch hergestellte Tumorproteine oder rekombinante Proteine die Tumorantigene, oder Teile davon mit Rezeptoragonisten verbinden, die die Aufnahme in die DZ vermitteln, können ebenfalls verwendet werden. Diese Methoden zur Antigenbeladung können im unreifen und/oder im reifen Stadium der DZ angewendet werden.
Eine mögliche Strategie zur Herstellung von antigenspezifischen zytotoxischen T-Zellen zur autologen oder allogenen adaptiven T-Zell-Therapie umfasst die Herstellung von NFkappaB-aktivierten DZ, wie im Beispiel beschrieben, die Isolation von T-Zellen aus frischem oder kryokonserviertem Patentenmaterial einschließlich aber nicht beschränkt auf Blut oder Blutzellen oder anderem Gewebe des Patienten, ferner die antigenspezifische Vermehrung dieser T-Zellen durch wiederholte Inkubation mit den NFkB-aktivierten DZ, die mit dem entsprechenden Antigen beladen sind, die Kryokonservierung der T-Zellen in geeigneten Portionen, Kontrolle der Qualität der T-Zellen durch Bestimmung ihrer antigenspezifischen lytischen Aktivität und ihrer Fähigkeit zur antigenspezifischen Zytokinsekretion, die intravenöse, intratumorale, intraperitoneale oder andere Injektion der T-Zellen in den Patienten in einer oder in mehreren gestaffelten Gaben.
Sofern nicht anders definiert, haben die hier verwendeten Begriffe dieselbe Bedeutung wie im Stand der Technik.

**Sequenzprotokoll, Freier Text:**

| SEQ ID NO: | Beschreibung |
|---|---|
| 1 | Wild-Typ IKKα Protein |
| 2 | IKKα-EEA16/64A Protein (AS 1-24 TAG) |
| 3 | IKKα-EEA16/64A Nukleotidsequenz |
| 4 | Wild-Typ IKKβ-Protein |
| 5 | IKKβ-EEA10/64 Protein (AS 1-17 TAG) |
| 6 | IKKβ-EEA10/64 Nukleotidsequenz |
| 7 | IKKα-K44MA16/64A Protein (AS 1-23 TAG) |
| 8 | IKKα-K44MA16/64A Nukleotidsequenz |
| 9 | IKKβ-K44MA10/64 Protein (AS 1-23 TAG) |
| 10 | IKKβ-K44MA10/64 Nukleotidsequenz |

### Beispiele

### Materialien und Methoden

Elektroporation der DZ: Reife oder unreife DZ wurden auf ca. 40-60 x 10⁶ Zellen/ml mit OptiMEM eingestellt (minimales Volumen für eine 4 mm Elektroporationsküvette: 100µl) und in die vorbereiteten Küvetten pipettiert. In die Küvette wurde in der Zwischenzeit RNA kodierend für IKKβ-EEA10, IKKα-EEA16 vorgelegt. Die Elektroporation erfolgte mit dem Programm *square-wave-pulse* bei 500V für 1 ms (4 mm Küvette). Unmittelbar nach Elektroporation wurden die DZ in vorbereitetes DZ-Medium (inkl. IL-4 und GM-CSF) gegeben und im Brutschrank für folgende Experimente inkubiert. Wurden die unreif transfizierten DZ nach der Elektroporation zur Reife gebracht, wurde dem DZ-Medium Reifungscocktail (IL1-ß, IL-6, TNFα und PGE₂) zugegeben.

### A: Sequenzen IKKα und IKKβ konstitutiv aktive Mutanten

1. Sequenz IKKα-EEA16/64A (SEQ ID NO:2): Vergleich Aminosäure-Sequenz IKKα-EEA16/64A (SEQ ID NO:2) mit Aminosäure-Sequenz Wild-Typ IKKα (SEQ ID NO:1): EE Mutationen (in Pos 200 und 204 von SEQ ID NO:2) verursachen konstitutive Aktivität von IKKα, A16 Mutationen (in Pos. 685, 686, 689, 693, 694, 700, 703, 704, 710, 711, 717, 719, 723, 729, 730, 745 und 746 von SEQ ID NO:2) entfernen destabilisierende Serine und Threonine, was zu einer stark erhöhten Stabilität des Proteins führt. Die entsprechende Nukleotidsequenz ist in SEQ ID NO:3 gezeigt.
2. Sequenz IKKβ-EEA10/64 (SEQ ID NO:5): Vergleich Aminosäure-Sequenz IKKβ-EEA10/64A (SEQ ID NO:5) mit Aminosäure-Sequenz Wild-Typ IKKβ (SEQ ID NO:4) EE Mutationen (in Pos 231 und 235 von Seq ID NO:5) verursachen konstitutive Aktivität von IKKβ, A10 Mutationen (in Pos. 724, 726, 729, 733, 736, 743, 746, 749, 751 und 759 von SEQ ID NO:2) entfernen destabilisierende Serine, was zu einer stark erhöhte Stabilität des Proteins führt. Die entsprechende Nukleotidsequenz ist in SEQ ID NO:6 gezeigt.

### B: Sequenzen IKKα und IKKβ hemmende Mutanten

3. Sequenz IKKα-K44MA16/64A (SEQ ID NO:7): Vergleich Aminosäure-Sequenz IKKα-K44MA16/64A (SEQ ID NO:7) mit Aminosäure-Sequenz Wild-Typ IKKα (SEQ ID NO:1). Die Kinaseaktivität wird durch einen Austausch der AS Lysin (Lys44; Pos. 67 in SEQ ID NO:7), in der ATP-Bindestelle, durch Methionin inhibiert. Durch Dimerisierung hat diese Mutante eine dominant negative Wirkung. A16 Mutationen (Pos. 684, 685, 688, 692, 693, 699, 702, 703, 709, 710, 716, 718, 722, 728, 729, 744 und 745 von SEQ ID NO:7) entfernen destabilisierende Serine und Threonine, was zu einer stark erhöhte Stabilität des Proteins führt. Die entsprechende Nukleotidsequenz ist in SEQ ID NO:8 gezeigt.
4. Sequenz IKKβ-K44MA10/64 (SEQ ID NO:9): Vergleich Aminosäure-Sequenz IKKβ-K44MA10/64A (SEQ ID NO:9) mit Aminosäure-Sequenz Wild-Typ IKKβ (SEQ ID NO:4)._Die Kinaseaktivität wird durch einen Austausch der AS Lysin (Lys44; Pos. 67 in SEQ ID NO:9), die ATP-Bindestelle, durch Methionin inhibiert. Durch Dimerisierung hat diese Mutante eine dominant negative Wirkung. A10 Mutationen (Pos. 693, 695, 698, 702, 705, 712, 715, 718, 720 und 728 von SEQ ID NO:9) entfernen destabilisierende Serine, was zu einer stark erhöhte Stabilität des Proteins führt. Die entsprechende Nukleotidsequenz ist in SEQ ID NO:10 gezeigt.

Beispiel 1: Sekretion von IL-12p70 und IL-10 durch IKKβ-EEA10-RNA-elektroporierte dendritische Zellen.
Dendritische Zellen wurden unreif (iDC) oder reif (mDC) ohne RNA, mit einer Kontroll-RNA oder der IKKβ-EEA10-RNA (SEQ ID NO:6) elektroporiert. Die Hälfte der unreif elektroporierten Zellen wurden unmittelbar nach Elektroporation gereift (iDCm). Vierundzwanzig Stunden nach Elektroporation wurden die Zytokinkonzentrationen (IL-12p70 und IL-10) in den Überständen in einem *cytometric bead array* (CBA) bestimmt. Die in Fig. 2(a) bzw. (b) gezeigten Daten stellen ein repräsentatives von vier unabhängigen Experimenten dar.

Beispiel 2: Expression von Oberflächenmarkern auf dendritischen Zellen, transfiziert mit der NFκB-Signalweg-Komponente IKKß-EEA10. Unreife (iDC) und reife (mDC) dendritische Zellen wurden mit RNA, die für IKKß-EEA10 (SEQ ID NO:6) kodiert, elektroporiert. Die Hälfte der unreif elektroporierten Zellen wurden nach der Elektroporation mit Reifungscocktail versetzt (iDCm). Als Kontrollkonditionen wurden DZ ohne RNA oder mit irrelevanter RNA (Kontroll-RNA) elektroporiert. Die DZ wurden nach der Elektroporation 24 h in DZ-Medium kultiviert, geerntet und mit einem PE-markierten Antikörpern gegen CD40, CD80 und CD70 gefärbt. Die PE-Markierung bezeichnet die Kopplung zwischen dem Pigment Phycoerythrin und einem Antikörper. Die durchschnittliche Fluoreszenzintensität (MFI) der elektroporierten dendritischen Zellen wurde über Durchflusszytometrie bestimmt. Die in Fig. 3 angegebenen Werte zeigen die spezifische MFI, welche aus der gemessenen relativen Fluoreszenz abzüglich der gemessenen Fluoreszenz des Isotypantikörpers berechnet wurde. Die Daten stellen ein repräsentatives von vier unabhängigen Experimenten dar.

Beispiel 3: Tetramerfärbung der Stimulation autologer T-Zellen mit dendritischen Zellen, elekroporiert mit RNA von NFκB-Signalweg-Komponenten.

Reife dendritische Zellen wurden mit Kontroll-RNA, IKKß-EEA10-RNA (SEQ ID NO:6) und IKKα-EE-RNA, oder mit einer Kombination aus IKKß-EEA10- und IKKα-EE-RNA elektroporiert. Ein Teil der Zellen wurde mit RNA, die für den Tumor-Marker MelanA kodiert, co-elektroporiert (+ MelanA-RNA). Drei Stunden nach Elektroporation wurde eine Hälfte der Konditionsreihe ohne MelanA-RNA mit MelanA/A2-Peptid für 1 h beladen (+ Peptidbeladung). Vier Stunden nach Elektroporation wurden autologe CD8⁺ T-Zellen mit den dendritischen Zellen im Verhältnis 10:1 stimuliert. Nach einer Woche wurden die Anzahl antigenspezifischer T-Zellen analysiert und deren Phänotyp durch CCR7- und CD45RA-Färbung bestimmt. Die T-Zellen wurden nach einem Priming Fig. 4(a) und nach einer Restimulation Fig.4(b) analysiert. Die Abbildungen zeigen Daten von einem Spender.

Beispiel 4: Migration reifer dendritischer Zellen 24h nach RNA-Transfektion mit NFκB-Signalweg-Komponenten.
Reife DZ wurden mit RNA kodierend für GFP, IKKß-EEA10 (SEQ ID NO:6) und IKKα-EE allein, und in Kombination, elektroporiert. Die DZ wurden nach der Elektroporation für 24 h kultiviert und anschließend auf ihre Migrationsfähigkeit für 2 h in einem Transwell-Assay getestet. Die Ergebnisse sind in Fig. 5 gezeigt (Kondition ohne Chemokin (=neg); Chemokin im Insert (=anti); Chemokin in der Vertiefung (=zu)). Die gezeigten Daten stellen Mittelwerte mit der Standartabweichung von drei unabhängigen Experimenten dar.

Beispiel5: Verbesserung von DZ durch RNA-Transfektion mit NFκB-Mutanten.
Stimulierung von DZ mit Komponenten des NFκB-Signalwegs, IKKβ-EE-A10 und IKKα-EE-A16 (SEQ ID NO 3): Es wurden die folgenden Konstrukte verwendet: IKKβ-EE-A10 stimuliert den klassischen NFκB-Signalweg, der zur Aktivierung und Reifung der DZ führt, und IKKα-EE-A16 ist ein Aktivator des alternativen NFκB-Signalwegs.
Die Wirkungen der Elektroporation mit IKKβ-EE-A10 waren hauptsächlich eine Hochregulierung von Oberflächenmarkern (CD25, CD40, CD70, CD80, CD83 und OX-40L, Figuren 6-8) und eine Hochregulierung der Cytokinsekretion, insbesondere von IL12p70, während IL-10 in sehr geringen Mengen sezerniert wurde (Figur9). Andere sezernierte Cytokine waren: IL-6, TNFα (Figur 12), IL-8 und IL-1β (Figur11). Diese Wirkungen wurden verstärkt, als RNA der Aktivatoren des klassischen und des alternativen NFκB-Signalwegs (IKKβ-EE-A10 und IKKα-EE-A16) coelektroporiert wurden (Figuren 6-11). Die Elektroporation von IKKα-EE-A16-RNA allein hatte vergleichbare Wirkungen wie geringfügig kleinere Mengen der sezernierten Cytokine und die Expression von Oberflächenmarkern (Figur 6-11). Die elektroporierten mDZ zeigten besonders nach der dritten Stimulation eine viel größere stimulatorische Kapazität gegenüber autologen T-Zellen (Figur12). DZ, die mit nur einem Aktivator (IKKα-EE-A16 oder IKKβ-EE-A10) elektroporiert wurden, hatten ähnliche stimulatorische Kapazitäten (bis zu dreifach im Vergleich zum Kontrollzustand MelA), während die DZ, die mit beiden Aktivatoren elektroporiert wurden, die höchste Kapazität zur Stimulation von spezifischen T-Zellen hatten (Expansion spezifischer T-Zellen auf das Siebenfache).
Dosisabhängigkeitsexperimente wurden durchgeführt, um die beste Menge an RNA zu bestimmen, die während der RNA-Transfektion von DZ verwendet werden sollte. Reife DZ wurden mit steigenden RNA-Konzentrationen elektroporiert. Steigende Expressionsmuster von Oberflächenmarkern (CD25, CD40, CD70 und OX-40L) wurden je nach der Konzentration der transfizierten RNA erhalten (Figur13). Aber dennoch führte der Zustand der Elektroporation mit beiden Aktivatoren, IKKβ-EE-A10 und IKKα-EE-A16 (jeweils 15 µg), zu einer erhöhten Expression aller Marker, insbesondere CD70, im Vergleich zu 30 µg RNA von einem Aktivator allein.
Bei der Cytokinsekretion wurde eine dosisabhängige Hochregulation erhalten, insbesondere von IL12p70, während IL-10 in einer sehr niedrigen Menge sezerniert wurde (Figur14). Außerdem war die Sekretion von IL-6, IL-8 und TNF dosisabhängig mit einem ähnlichen Muster (Daten nicht gezeigt). Hier war die Menge an sezernierten Cytokinen nicht höher, wenn RNA der Aktivatoren des klassischen und des alternativen NFκB-Signalwegs (IKKβ-EE-A10 und IKKα-EE-A16) coelektroporiert wurden (vergleiche 30 µg der einzelnen RNA mit einer Kombination von 15 µg RNA von beiden Aktivatoren).

Beispiel6: NFκB-Aktivität bei transfizierten 293T-Zellen: 293T-Zellen wurden mit Aktivatoren beider NFκB-Signalwege elektroporiert und mit einem Vektor, der für Luciferase unter der Kontrolle eines NFκB-Promotors codierte, mit coelektroporiert. In allen Fällen (IKKα-EE-A16 und IKKβ-EE-A10 allein oder in Kombination) wurde die Luciferase-Aktivität 24 h nach der Elektroporation gemessen (Figur15). Wiederum zeigte der Fall der RNA-Transfektion mit beiden Aktivatoren die größte Wirkung.
Dieser Assay wurde auch bei DZ durchgeführt, führte jedoch zu keinem Ergebnis (Daten nicht gezeigt).
IKKβ-K44M-A10 und IKKα-K44M-A16: Mit einem Inhibitor des klassischen (IKKβ-K44M-A10) und des alternativen NFκB-Signalwegs (IKKα-K44M-A16) wurde ein Luciferase-Assay mit 293T-Zellen durchgeführt, die mit IKKα-K44M-A16- oder IKKβ-K44M-A10-RNA allein oder in Kombination elektroporiert und mit Luciferase-Vektoren, die einen NFκB-Promotor umfassen, coelektroporiert wurden. Die NFκB-Signalwege der transfizierten 293T-Zellen wurden über Nacht mit löslichem CD40L aktiviert. Die Luciferase-Aktivität wurde 24 h nach der Elektroporation gemessen. Beide Inhibitoren waren eindeutig in der Lage, die Luciferase-Aktivität im Vergleich zur positiven Kontrolle, die nur mit Luciferase-Vektor transfiziert und mit löslichem CD40L aktiviert wurde, zu verringern (Figur15).

Beispiel7: Sekretion von IL-12p70 in reifen dendritischen Zellen, die mit RNA transfiziert wurden, die konstitutiv aktive IKK Mutanten kodiert.
Aus Monozyten gewonnene dendritische Zellen wurden an Tag 6 mit Hilfe des Standard-Reifungs-Cocktails (IL-1β, IL-6, TNFα und PGE2) für 24h zur Reife gebracht und anschließend elektroporiert. Transfiziert wurden die Zellen ohne RNA, mit RNA, die die konstitutiv aktive Mutanten IKKαEEA16 und IKKβEEA10 kodiert und einer Kombination beider RNAs (siehe Fig.16). Anschließend wurde die Konzentration von IL-12p70 im Medium 4h, 24h und 48h nach der Elektroporation gemessen. Hier wurde die die Produktion von IL12p70 über einen Zeitraum von 2 Tagen beobachtet. Die Verwendung beider Mutanten führte zur höchsten IL-12p70-Produktion. Ein repräsentatives Experiment von dreien ist gezeigt.

Beispiel8: Migration von reifen dendritischen Zellen, die mit RNA transfiziert wurden, die konstitutiv aktive IKK Mutanten kodiert. Aus Monozyten gewonnene dendritische Zellen wurden an Tag 6 mit Hilfe des Standard-Reifungs-Cocktails (IL-1β, IL-6, TNFα und PGE2) für 24h zur Reife gebracht und anschließend elektroporiert. Transfiziert wurden die Zellen mit 5 µg/100 µl RNA, die MelanA kodiert und mit 15 µg/100 µl RNA, die konstitutiv aktive Mutanten von IKKα und IKKβ kodiert und einer Kombination beider RNAs kodiert (siehe Fig. 17). Anschließend wurde die Migrationsfähigkeit der transfizierten Zellen zum Chemokin CCL19 untersucht. Die Ergebnisse sind in Fig. 17 gezeigt (Kondition ohne Chemokin (=neg); Chemokin im Insert (=anti); Chemokin in der Vertiefung (=zu)). Mittelwerte mit Standardfehlern aus 4 unabhängigen Experimenten sind gezeigt

## Patentansprüche

1. Dendritische Zellen (DZ), die durch RNA-Transfektion mit einer oder mehreren Nukleotidsequenzen, die für mindestens ein mutiertes signalübertragendes Protein des NFκB-Signalweges kodieren, in ihrem NFκB-Signalweg manipuliert sind, wobei das mutierte signalübertragende Protein des NFκB-Signalweges eine konstitutiv-aktive IKKα oder IKKβ Mutante ist, wobei
(i) die konstitutiv-aktive IKKα Mutante die Aminosäurereste 25 bis 769 von SEQ ID NO:2 umfasst; oder
(ii) die konstitutiv-aktive IKKβ Mutante die Aminosäurereste 18 bis 773 von SEQ ID NO:5 umfasst.

2. DZ nach Anspruch 1, wobei die konstitutiv-aktiven IKKα und IKKβ Mutanten die Sequenz von SEQ ID NO:2 bzw. SEQ ID NO:5 aufweisen, oder die RNA Sequenz die SEQ ID NO:3 oder 6 aufweisen.

3. DZ nach Anspruch 1 oder 2, wobei die DZ
(i) reife DZ sind; und/oder
(ii) NFκB-aktivierte DZ sind, die IL-12p70 produzieren; und/oder
(iii) NFκB-aktivierte DZ sind, die IL-10 produzieren; und/oder
(iv) weiterhin mit einem oder mehreren Targetantigenen beladen sind.

4. Verfahren zur ex-vivo Herstellung von NFκB-Signalweg manipulierten DZ wie in Anspruch 1 bis 3 definiert, umfassend die RNA-Transfektion von unreifen oder reifen DZ mit einer oder mehreren Nukleotidsequenzen, die für ein mutiertes signalübertragendes Protein des NFκB-Signalweges wie in Anspruch 1 bis 3 definiert kodieren.

5. Verfahren nach Anspruch 4, wobei die RNA-Transfektion durch Elektroporation erfolgt.

6. Verfahren nach Anspruch 4 oder 5, das weiterhin
(i) im Falle der RNA-Transfektion von unreifen DZ, das Behandeln mit einem Reifungsstimulus; und/oder
(ii) das Beladen der DZ mit einen Targetantigen; und/oder
(iii) die Kryokonservierung von reifen DZ
umfasst.

7. Zusammensetzung, pharmazeutische Zusammensetzung oder Medikament umfassend DZ nach einem oder mehreren der Ansprüche 1 bis 3 und optional pharmazeutisch geeignete Hilfs- und Trägerverbindungen, wobei vorzugsweise die DZ autologe DZ sind.

8. Verwendung der DZ nach einem oder mehreren der Ansprüche 1 bis 3 zur Stimulation autologer CD8+ T-Zellen ex vivo.

9. Verwendung nach Anspruch 8, wobei die NFκB-aktivierten DZ für passive T-Zell-Übertragung und Erzeugung eines T-Zell-Klons geeignet sind.

10. DZ nach einem oder mehreren der Ansprüche 1 bis 3 zur Verwendung in der Behandlung von Erkrankungen in einem Patienten.

11. DZ zur Verwendung nach Anspruch 10, wobei die NFκB-aktivierten DZ zur DZ-basierten Impfung, zur therapeutischen Impfung gegen Krebs oder Infektionskrankheiten und als präventiver Impfstoff geeignet sind.

12. Verfahren zur Expansion von T Zellen einschließlich der Stimulation autologer CD8+ T-Zellen ex vivo, umfassend Stimulieren der T Zellen mit DZ nach einem oder mehreren der Ansprüche 1 bis 3.

13. Verfahren nach Anspruch 12, wobei die NFκB-aktivierten DZ zur T-Zell-Expansion für T Zellen zur passive T-Zell-Übertragung und Erzeugung eines T-Zell-Klons geeignet sind.

## Claims

1. Dendritic cells (DCs), the NFκB signaling pathway of which has been manipulated by RNA transfection with one or more nucleotide sequences encoding at least one mutant signal transducing protein of the NFκB signaling pathway, wherein the mutant signal transducing protein of the NFκB signaling pathway is a constitutively active IKKα or IKKβ mutant, wherein
(i) the constitutively active IKKα mutant comprises the amino acid residues 25 to 769 of SEQ ID NO:2; or
(ii) the constitutively active IKKβ mutant comprises the amino acid residues 18 to 773 of SEQ ID NO:5.

2. The DCs according to claim 1, wherein the constitutively active IKKα and IKKβ mutants have the sequence of SEQ ID NO:2 and SEQ ID NO:5, respectively, or have the RNA sequence of SEQ ID NO:3 or 6.

3. The DCs according to claim 1 or 2, wherein said DCs
(i) are mature DCs; and/or
(ii) are NFκB-activated DCs that produce IL-12p70; and/or
(iii) are NFκB-activated DCs that produce IL-10; and/or
(iv) are further loaded with one or more target antigens.

4. Method for ex vivo manufacture of DCs, the NFκB signaling pathway of which has been manipulated, as defined in claims 1 to 3, comprising the RNA transfection of immature or mature DCs with one or more nucleotide sequences encoding a mutant signal transducing protein of the NFκB signaling pathway as defined in claims 1 to 3.

5. The method according to claim 4, wherein the RNA-transfection is carried out by electroporation.

6. The method according to claim 4 or 5, further comprising
(i) in the case of the RNA-transfection of immature DCs, treating with a maturation stimulus; and/or
(ii) loading of the DCs with a target antigen; and/or
(iii) the cryopreservation of mature DCs.

7. A composition, pharmaceutical composition or medicament comprising DCs according to one or more of claims 1 to 3, and optionally pharmaceutically acceptable excipients and carrier compounds, wherein preferably said DCs are autologous DCs.

8. Use of the DCs according to one or more of claims 1 to 3 for the stimulation of autologous CD8+ T cells ex vivo.

9. Use of claim 8, wherein the NFκB-activated DCs are suitable for passive T cell transfer and generation of a T cell clone.

10. DCs according to one or more of claims 1 to 3 for use in the treatment of diseases in a patient.

11. DCs for use of claim 10, wherein the NFκB-activated DCs are suitable for DC based vaccination, for the therapeutic vaccination against cancer or infectious diseases and as a preventive vaccine.

12. Method for the expansion of T cells, including the stimulation of autologous CD8+ T cells ex vivo, comprising stimulating the T cells with DCs according to one or more of claims 1 to 3.

13. Method of claim 12, wherein the NFκB-activated DCs are suitable for T-cell expansion of T cells for passive T cell transfer and generation of a T cell clone.

## Revendications

1. Cellules dendritiques (CD), dont la voie de signalisation NFκB a été manipulée par transfection de leur ARN avec une ou plusieurs séquences nucléotidiques, qui codent pour au moins une protéine de transmission du signal de la voie de signalisation NFκB mutée, ladite protéine de transmission du signal de la voie de signalisation NFκB mutée étant un mutant IKKα ou IKKβ constitutivement actif, dans laquelle
(i) le mutant IKKα constitutivement actif comprend les résidus d'acides aminés 25 à 769 de la SEQ ID No: 2 ; ou
(ii) le mutant IKKβ constitutivement actif comprend les résidus d'acides aminés 18 à 773 de la SEQ ID No: 5,

2. Cellules dendritiques selon la revendication 1, dans lesquelles les mutants IKKα ou IKKβ constitutivement actifs présentent la séquence de SEQ ID No: 2 ou de SEQ ID No: 5, ou la séquence ARN de la SEQ ID No: 3 ou 6.

3. Cellules dendritiques selon la revendication 1 ou 2, dans lesquelles les CD
(i) sont des cellules dendritiques matures; et/ou
(ii) sont des cellules dendritiques activées par NFκB, qui produisent IL-12p70; et/ou
(iii) sont des cellules dendritiques activées par NFκB, qui produisent IL-10; et/ou
(iv) sont en outre chargées avec un ou plusieurs antigènes cibles.

4. Procédé de préparation ex-vivo de cellules dendritiques dont la voie de signalisation NFκB a été manipulée comme défini dans les revendications 1 à 3, comprenant la transfection de l'ARN des cellules dendritiques immatures ou matures avec une ou plusieurs séquences nucléotidiques qui codent pour une protéine mutée de transmission du signal de la voie de signalisation NFκB comme défini dans les revendications 1 à 3.

5. Procédé selon la revendication 4, dans lequel la transfection de l'ARN s'effectue par électroporation.

6. Procédé selon la revendication 4 ou 5, qui comprend en outre
(i) dans le cas de la transfection de l'ARN de cellules dendritiques immatures, le traitement avec un stimulus de maturation; et/ou
(ii) le chargement des cellules dendritiques avec un antigène cible; et/ou
(iii) la cryoconservation des cellules dendritiques matures.

7. Composition, composition pharmaceutique ou médicament comprenant les cellules dendritiques selon l'une ou plusieurs des revendications 1 à 3, et éventuellement des composés auxiliaires ou supports pharmaceutiquement appropriés, les cellules dendritiques étant des cellules dendritiques autologues.

8. Utilisation des cellules dendritiques selon l'une ou plusieurs des revendications 1 à 3 pour la stimulation des cellules T CD8+ autologues ex vivo.

9. Utilisation selon la revendication 8, dans laquelle les cellules dendritiques activées par NFκB sont appropriées au transfert passif des cellules T et à la production d'un clone de cellules T.

10. Cellules dendritiques selon l'une ou plusieurs des revendications 1 à 3 pour l'utilisation dans le traitement de maladies chez un patient.

11. Cellules dendritiques selon la revendication 10, dans lesquelles les cellules dendritiques activées par NFκB sont appropriées pour la vaccination à base de cellules dendritiques, pour la vaccination thérapeutique contre le cancer ou les maladies infectieuses et comme vaccin préventif.

12. Procédé d'expansion de cellules T, y compris la stimulation de cellules T CD8+ autologues ex vivo, comprenant la stimulation des cellules T avec des cellules dendritiques selon l'une ou plusieurs des revendications 1 à 3.

13. Procédé selon la revendication 12, dans lequel les cellules dendritiques activées par NFκB destinées à l'expansion des cellules T sont appropriées pour des cellules T pour le transfert passif des cellules T et la production d'un clone de cellules T.
